# EUROPEAN PATENT SPECIFICATION

(11) **EP 3 904 340 B1**
(45) Date of publication and mention of the grant of the patent: **05.07.2023**
(21) Application number: 19902141.1
(22) Date of filing: 18.09.2019
(51) Int. Cl.: C07D 211/42, C07D 401/06

(54) **SYNTHESIS METHOD FOR HALOFUGINONE AND INTERMEDIATE THEREOF**
SYNTHESEVERFAHREN FÜR HALOFUGINON UND ZWISCHENPRODUKT DAVON
PROCÉDÉS DE SYNTHÈSE D'UNE HALOFUGINONE ET D'UN INTERMÉDIAIRE DE CELLE-CI

(30) Priority: 28.12.2018 CN 201811626095; 30.01.2019 CN 201910090473
(43) Date of publication of application: 03.11.2021
(73) Proprietor: Launch-Pharma Technologies, Ltd., Guangzhou, Guangdong 510530 (CN)
(72) Inventor: JIN, Yehua, Guangzhou, Guangdong 510530 (CN); QIU, Fayang, Guangzhou, Guangdong 510530 (CN); XU, Hua, Guangzhou, Guangdong 510530 (CN); YIN, Wenhao, Guangzhou, Guangdong 510530 (CN)
(74) Representative: Haseltine Lake Kempner LLP
(86) International application number: PCT/CN2019/106485
(87) International publication number: WO 2020/134212

(56) References cited:
- CN-A- 1 583 729
- CN-A- 101 648 942
- CN-A- 101 987 843
- CN-A- 101 987 843
- CN-A- 103 275 063
- CN-A- 103 275 063
- CN-A- 103 467 449
- Hua Xu et al: "A Scalable Total Synthesis of Halofuginone", Organic Process Research & Development, vol. 23, no. 5, 6 March 2019 (2019-03-06), pages 990-997, XP055715565, ISSN: 1083-6160, DOI: :10.1021/acs.oprd.9b00059

## Description

### CROSS-REFERENCE TO RELATED APPLICATION

This application is a continuation of international PCT application serial no. PCT/CN2019/106485, filed on September 18, 2019, which claims the priority benefit of China application no. 201811626095.2 filed on December 28, 2018, and China application no. 201910090473.8, filed on January 30, 2019.

### Technical Field

The present disclosure relates to the chemical synthesis technology, in particular to a synthesis method for halofuginone and halofuginone intermediates.

### Background Art

Halofuginone (Formula 1) is a halogenated derivative of natural product febrifugine, also known as bromochloro-halofuginone, chloroquinone, halofuginone. Halofuginone is a new type of broad-spectrum anticoccidial drug, which has strong inhibitory effect on many kinds of chicken coccidiosis. Halofuginone can obviously control the symptoms of coccidiosis and completely inhibit the discharge of oocysts (except for *Eimeria cumulata*) after use, thereby no longer polluting the environment and reducing the possibility of reinfection. Halofuginone can inhibit and kill the sporozoites in the three stages in the development cycle, namely: sporozoites, the first and second generation schizoites. Halofuginone has a unique chemical structure, so it has no cross-resistance with other existing anticoccidial drugs.

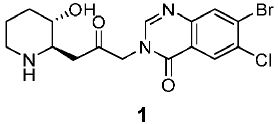

In addition, it is found that halofuginone specifically inhibits fibroblasts to produce type I collagen fibers, which prevents liver fibrosis, pulmonary fibrosis, scleroderma, and other diseases characterized by excessive synthesis of type I collagen. The inhibition of collagen synthesis leads to decreased in cell activity that play a key role in tumor growth, which in turn affects vascular growth and cell proliferation. It blocks the growth and metastasis of tumor cells. Therefore, the research of halofuginone in the treatment of malignant tumors entered the clinical trial stage approved by FDA in 2000. In 2002, halofuginone was approved in Europe for the treatment of systemic sclerosis.

The piperidine ring structure of febrifugine is unstable, so febrifugine and iso- febrifugine can be converted into each other through the process of "retro-conjugate- conjugate addition", which becomes one of the main strategies for the total synthesis of febrifugine. For example, in 2001, Yasuo Takeuchi reported the isomerization of isofebrifugine and febrifugine (as shown in equation I). When isofebrifugine was heated in various solvents, it could be converted into febrifugine, and the reaction reached equilibrium, indicating febrifugine was successfully synthesized from isofebrifugine (Tetrahedron, 2001, 57, 1213-1218).

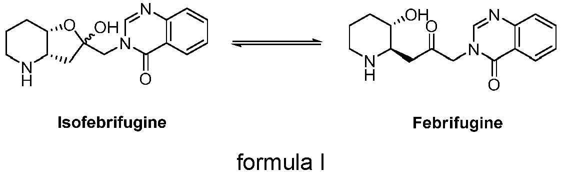

Halofuginone is a halogenated derivative of febrifugine, with only two hydrogens of the aromatic ring replaced by bromine and chlorine in chemical structure, while the piperidine ring is the same as febrifugine. It was found that halofuginone (1) and isofebrifugine (13) also have the property of mutual transformation. (Tetrahedron, 2017, 73, 5493-5499, as shown in equation II). Using this property, halofuginone was successfully synthesized from iso-halofuginone (CN201010257723.1, CN201310243084.7).

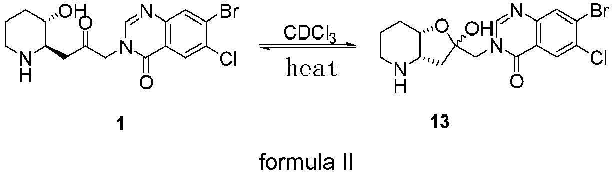

According to the structural characteristics and inverse synthesis analysis of halofuginone, it can be divided into piperidine ring side chain and quinazoline fragment. The quinazoline fragment can be connected with three types of piperidine ring intermediates (A, B, C) to synthesize halofuginone (as shown in equation III). These docking methods and detailed synthesis routes were reported by Paul Evans et al in the review article on the synthesis of halofuginone [Bioorganic & Medical Chemistry, 2018, 26 (9): 2199-2220].

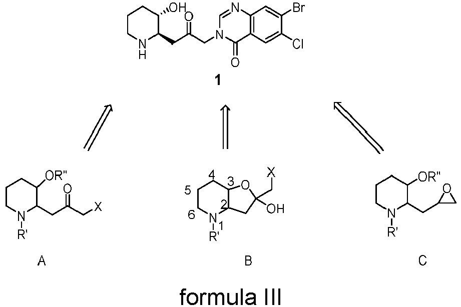

As mentioned above, the use of the interconvertible nature of halofuginone and isofebrifugine is one of the main strategies for the total synthesis of halofuginone, because it is easier to construct when the side chains of hydroxyl and carbonyl groups on the piperidine ring of febrifugine are in CIS structure. And further, when the side chains of hydroxyl and carbonyl groups are in CIS structure, they can form a hemiketal structure to reduce the protective steps of hydroxyl groups. B-type intermediates are the key intermediates of this synthesis strategy.

The key step in the synthesis of type B intermediates is how to introduce the 2-position side chain (G or H) in piperidine ring. There are mainly two methods for introducing the 2-position side chain in piperidine ring in the literature. One is through the allyl alkylation of N-protected piperidinone (D) (Chemical and Pharmaceutical Bulletin, 1999, 47, 905-906; Synthesis, 1999, 10, 1814-1818; Journal of Medicinal Chemistry, 2003,46, 4351-4359; ACS Medicinal Chemistry Letters, 2014, 5, 572-575; Patents US 20080004287, CN201010257723.1, CN201310243084.7). The other method is to synthesize intermediate E from 3-hydroxypyridine (F) through multi-step reaction, then subjected to allyl migration rearrangement reaction (Journal of medical chemistry, 2003, 46, 4351-4359; Chemical and Pharmaceutical Bulletin, 1999, 47, 905-906; Synthesis, 1999, 10, 1814-1818), as shown in equation IV below.

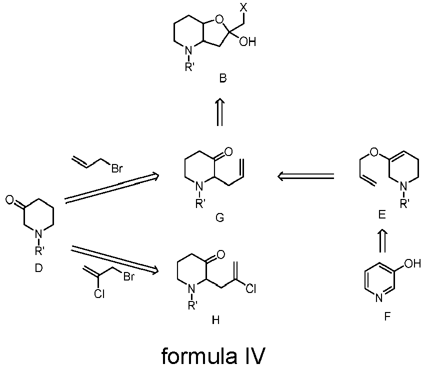

The common disadvantage of the above-described two methods is poor selectivity. If the same side chain is introduced at other positions on the piperidine ring, it can lead to impurities with similar structure, which is difficult for separation and purify as physicochemical properties are similar due to the similar structure.

In addition, by using this total synthesis method reported by this strategy, methoxyformyl, ethoxyformyl, tert butoxyformyl (BOC), benzyloxyformyl (CBZ), trichloroethoxyformyl (TROC) or benzyl (BN) are selected as the protective groups for the piperidine ring nitrogen atoms. These protective groups can be removed under strong acid, strong base or reduction conditions to prepare the target product, which is disadvantageous to the unstable property of halofuginone. As a result, the yield is low, the product quality is not good, or the purification is complicated.

In the literature reported by Takeuchi, Y et al., compound I reacted with boron trifluoride ether to form furan compound J (Heterocycles, 2000, 53, 2247-2252). We also encountered similar problems in the development of the synthesis routes. Racemic compound K was deprotected by TMSI, and produced compound L with a similar structure, as shown in formula V. It is concluded that the removal of protective groups with lewis acid mainly produces furan ring compounds (such as by-products J and L).

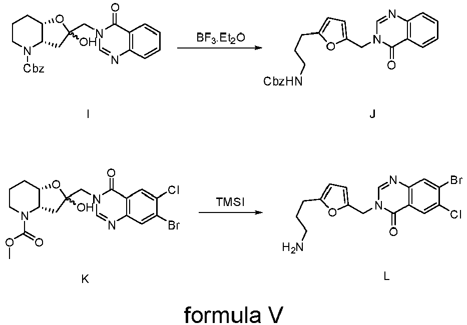

In the literature reported by Maiden T. M. M. et al. (Org. Biomol. Chem., 2018, 16, 4159), the Cbz protecting group of compound M was removed with hydrobromic acid, only 16% of the target product compound N was obtained, and the main product of deprotection is compound O (formula VI).

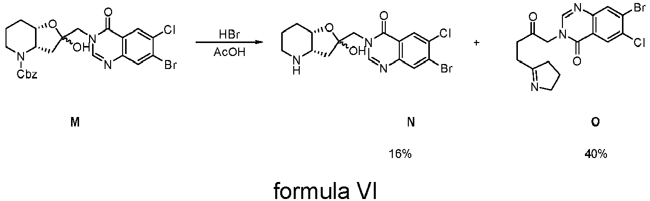

The authors of the article speculated that the production mechanism of the by-product was shown in formula VII.

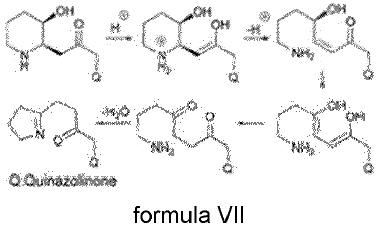

In the process of route development, we also tried to remove the protective group of racemic compound K with hydrochloric acid or hydrobromic acid, and obtained almost the same result as above. As shown in formula VIII, only product P was obtained within 0.5h, and product O was obtained by prolonging the reaction time, while the target product N was not detected. It is concluded that the deprotection with protonic acid mainly produces piperidine ring opening by-products and imine by-products (such as by-products P and O).

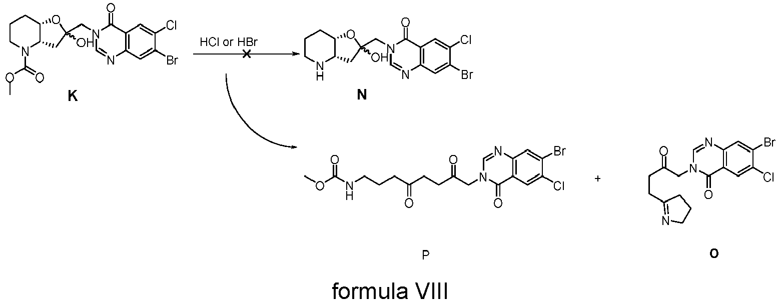

Similarly, the desired result can not be achieved by removing the protective group in the strong alkali condition. For example, in the literature reported by Laurence E. Burgess (Tetrahedron Letters, 1996, 37, (19), 3255-3258), compound Q was used to remove the ethoxyl group on the piperidine ring with potassium hydroxide to obtain the target product compound R, but the yield was only 10%, as shown in formula IX.

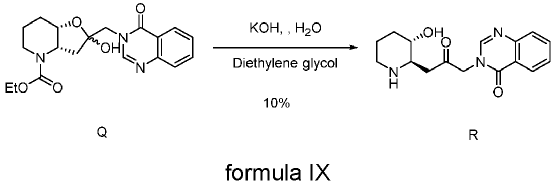

In some patents and literatures, the protective group of nitrogen on piperidine ring is removed by reduction. For example, in patents CN201010257723.1 and CN201310243084.7, zinc powder and acetic acid were used to remove Trichloroethoxyformyl (Troc) protected racemic compound S. When this experiment was repeated, we found that although there were no side reactions mentioned above, the bromine atoms on the aromatic ring were also reduced, forming racemic compound T. Similarly, we also tried hydrogen deprotection of racemic compound M catalyzed by palladium, and the reductive deprotection of bromine atoms also occurred to form racemic compound T, as shown in formula X.

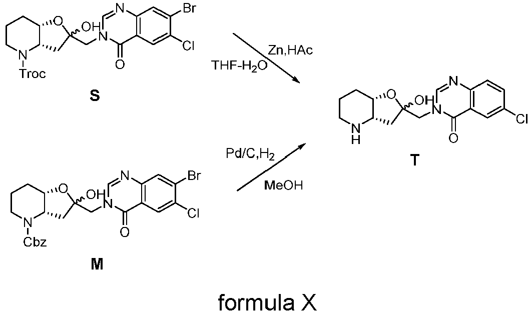

There are many problems in the synthesis route even when using compound A, C or other types of intermediates, such as low total yield, harsh reaction conditions, expensive raw materials, and the need for column chromatography to separate and purify.

Therefore, the products prepared by the existing synthesis methods are difficult to meet the quality standards of halofuginone, and cannot meet the huge market demand for halofuginone, let alone meet the declaration requirements of ICH.

In addition, the absolute configuration of halofuginone is *2R*, *3S,* and the optical rotation is "+", which means it has right-handed optical activity. However, halofuginone, a derivative of febrifugine, is used in veterinary medicine as a racemate without optical activity. In recent years, many studies on halofuginone have shown that the pharmacological activity of D-*2R*, *3S-*halofuginone with the same absolute configuration as natural product halofuginone is obviously better than that of L-*2S*, *3R-* halofuginone. Therefore, the synthesis of halofuginone with high optical activity is of great significance.

The piperidine ring segments of halofuginone and febrifugine contain two chiral centers in trans form, at 2-and 3-position. The reported chiral synthesis methods mainly include the following: (1) constructing the 2- and 3-position chiral centers by Sharpless asymmetric dihydroxylation or Sharpless epoxidation. (2) introducing the 3-position chiral central hydroxyl group through the reduction of carbonyl group by yeast. (3) introducing 3-position chiral hydroxyl group by acetylase. (4) constructing 3-hydroxy chirality through asymmetric aldol condensation catalyzed by chiral small molecule compounds. (5) Chemical resolution by brucine. (6) synthesis methods using chiral compounds as starting materials, etc. There are many disadvantages of asymmetric synthesis of optically active halofuginone or febrifugine, such as complicated synthesis route, harsh reaction conditions, low yield, complex separation and purification, and the use of highly toxic and expensive reagents.

### Summary of the Disclosure

Based on the above, the present disclosure provides a new synthesis method for halofuginone intermediate. The name of the intermediate is cis -2-(2- chloropropene) -3-hydroxypiperidine (with the structure shown in Formula 9), and the provided synthesis method for the intermediate has many advantages, such as simple process, few product impurities, no need of column chromatography purification, and high yield.
The detailed technical solution is as following.

A synthesis method for the halofuginone intermediate with the structure shown in Formula 9, comprising the following steps: 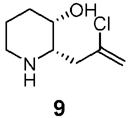
(a) alkylation reacting diethyl acetaminomalonate with 2,3-dichloropropene under the action of a base and a catalyst to form the compound of Formula 2;
(b) decarboxylation reacting the compound of Formula 2 in the presence of an acid catalyst to produce the compound of Formula 3;
(c) esterification reacting the compound of Formula 3 in the presence of an acid catalyst to produce the compound of Formula 4;
(d) nitrogen alkylation reacting the compound of Formula 4 with 4-halogenated butyrate under the action of a base and a catalyst, and then nitrogen protection reacting with amino protection reagent to produce the compound of Formula 5;
(e) Dieckmann condensation reacting the compound of Formula 5 under the action of the base to produce the compound of Formula 6;
(f) decarboxylation reacting the compound of Formula 6 in the presence of inorganic salt to produce the compound of Formula 7;
(g) reduction reacting the compound of Formula 7 under the action of a reducing agent to produce the compound of Formula 8;
(h) nitrogen deprotection reacting the compound of Formula 8 to produce the compound of Formula 9;
   the reaction formulas are as following: 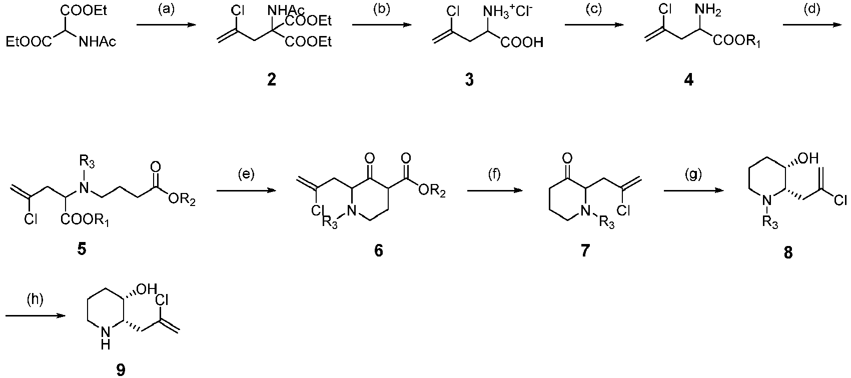
wherein,
R₁ is selected from: methyl, ethyl, propyl, isopropyl or tert-butyl; preferably, methyl and ethyl;
R₂ is selected from: methyl, ethyl;
R₃ is selected from: methoxyformyl, ethoxyformyl, tert-butoxyformyl, benzyloxyformyl, trichloroethoxyformyl or benzyl, preferably benzyloxyformyl.

The present disclosure also provides a preparation method for the halofuginone intermediate with optical activity, named as cis-2 - (2-chloropropenyl) - 3-hydroxypiperidine. The method has advantages such as simple process, less impurities, and no column chromatography purification. The method can be applied to preparation of halofuginone with optical activity and realize large scale production of halofuginone with high optical purity.

The detailed technical solution is as follows:
A preparation method of cis-2- (2-chloropropenyl)- 3-hydroxypiperidine with optical activity, comprises the following steps:
(1) In the first organic solvent, salt formation reacting the racemic cis-2-(2-chloropropenyl)-3-hydroxypiperidine with dibenzoyl tartaric acid or its derivatives to produce precipitate, and the precipitate is recrystallized to obtain a chiral double salt;
(2) In the second organic solvent, the chiral complex salt is neutralized to alkalinity with an alkaline aqueous solution to obtain cis-2-(2-chloropropenyl)-3-hydroxypiperidine with optical activity;

The racemic cis-2 - (2-chloropropenyl) - 3-hydroxypiperidine has the structure as shown in Formula (±)- 9; The optical active cis-2-(2-chloropropenyl)-3-hydroxypiperidine has the structure as shown in Formula (+) - 9 or Formula (-) - 9; The dibenzoyl tartaric acid or its derivative has the structure as shown in Formula 15 or Formula 16; The chiral double salt has the structure as shown in Formula 14 or Formula 17; The reaction equations are as following: 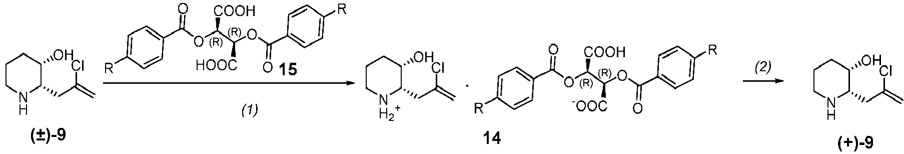 or, 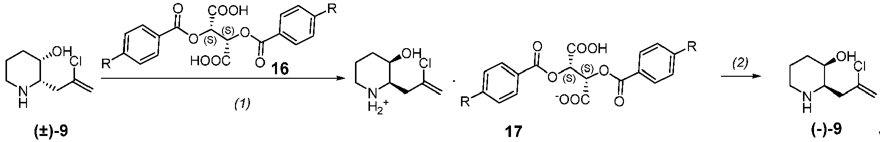 wherein, each R is independently selected from: hydrogen or C₁-C₄ alkoxy.

The present disclosure also provides a synthesis method of racemic halofuginone. This method has advantages such as simple process, less impurities, no column chromatography, and high yield.

The specific technical scheme is as follows:
A synthesis method for halofuginone with the structure of Formula 1, comprises the following steps: 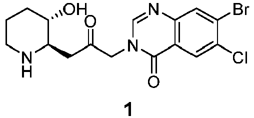
(i) reacting the compound of Formula 9 with an amino protection reagent under the action of alkali to produce a compound of Formula 10;
(j) reacting the compound of Formula 10 with olefin halogenation reagent and water, to produce a compound of Formula 11;
(k) reacting the compound of Formula 11 with the compound of Formula 12 under the action of a base, and then removing the 9-fluorenylmethoxyformyl protecting group on piperidine ring nitrogen, to produce a compound of Formula 13;
(l) isomerization reacting the compound of Formula 13 to produce a compound of Formula 1. 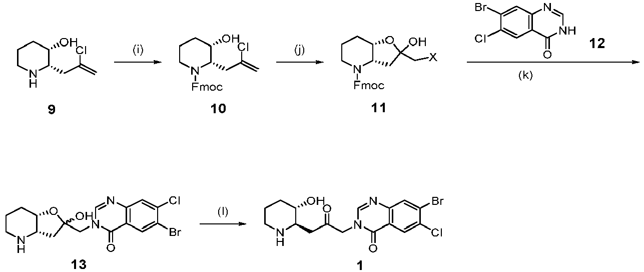
wherein, X is chlorine, bromine or iodine, preferably bromine.

The present disclosure also provides a synthesis method of halofuginone salt.

The detailed technical solution is as follows:
A synthesis method of halofuginone salt comprises the following steps: synthesizing halofuginone by the synthesis method for halofuginone descried above, then reacting the halofuginone with acid to obtain the halofuginone salt.

In some embodiments, the acid is hydrobromic acid, hydrochloric acid or lactic acid.

Understandably, in the above method for synthesizing halofuginone and halofuginone salt, the reaction products obtained from any step through step (a) to (l) can be used as raw materials to directly carry out subsequent reactions to synthesize halofuginone and its salt. For example, a compound of Formula 9 can be used as a raw material to synthesize halofuginone and halofuginone salts through step (i) to (l) as described above, or a compound of Formula 3 can be used as a raw material to synthesize halofuginone and halofuginone salt through step (c) to (l) as described above.

The present disclosure also provides a synthesis method for halofuginone with optical activity. The detailed technical solution is as follows.

A synthesis method for halofuginone with optical activity, comprising the following steps:
(3) reacting the cis-2 - (2-chloropropenyl) - 3-hydroxypiperidin with optical activity with an amino protecting agent in the presence of a base to form optically active compounds of Formula (+) - 10 or Formula (-) - 10;
(4) reacting the optically active compound of Formula (+) - 10 or Formula (-) - 10 with olefin bromination reagent and water to form optically active compound of Formula (+) - 11 or Formula (-) - 11;
(5) reacting the optically active compound of Formula (+) - 11 or Formula (-) - 11 with the compound of Formula 12 under the action of alkali, and then the 9-fluorenylmethoxyformyl protecting group on the piperidine ring nitrogen is removed to form the optically active compound of Formula (+) - 13 or Formula (-) - 13;
(6) The optically active compound of Formula (+) - 13 orFormula (-) - 13 is isomerized to produce the optically active halofuginone;
the optically active halofuginone has the structure as shown in Formula (+) - 1 or Formula (-) - 1, and the reaction formulas are as following: 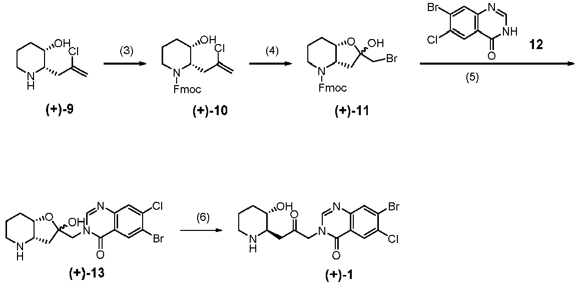 or

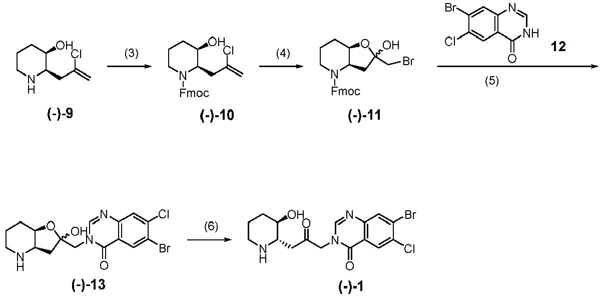

The present disclosure also provides a synthesis method of halofuginone salt with optical property.

The specific technical scheme is as following:
A synthesis method for halofuginone salt comprises the following steps: synthesizing halofuginone by the synthesis method for halofuginone with high optical property descried above, then reacting the halofuginone with acid to obtain the halofuginone salt with high optical property.

In some embodiments, said acid is hydrobromic acid, hydrochloric acid or lactic acid.

Understandably, in the above method for synthesizing optically active halofuginone, any reaction products obtained from step (a) to (g) and (1) to (6) can be used as raw materials to directly carry out subsequent reactions to synthesize optically active halofuginone. For example, the racemic cis-2 - (2-chloropropenyl) - 3-hydroxypiperidine can be used as the raw material to synthesize the optically active halofuginone through step (1) to (6) described above, or the compound of Formula 3 can be used as the raw material to synthesize the optically active halofuginone through step (c) to (g) and (1) to (6) described above.

The present disclosure discovers that the reasonable selection of the protective group of piperidine ring nitrogen atom is very important in the synthesis for halofuginone. If the protective group is not selected properly, many by-products will be produced in the deprotection process, which will lead to low yield of the product, poor product quality, or difficult for purification. In the present disclosure, 9-fluorenylmethoxyformyl (Fmoc) is selected as the protective group of piperidine ring nitrogen atom, and its removal is very simple, which can be removed only by reacting in the secondary or tertiary organic amine for a few seconds or minutes, thus greatly reduces the generation of by-products. In addition, the present disclosure uses a synthesis strategy that is completely different from the prior art to synthesize the halofuginone intermediate. The synthesis method for halofuginone and halofuginone intermedium of the present disclosure solves the technical defects in current halofuginone production with many advantages, such as simple process, low cost, fewer by-products in the synthesis process, simple purification process, no need column chromatography purification, high yield, fewer impurities, high purity, controllable product quality, and easy to meet the requirements of ICH declaration, which can be used for industrial production of halofuginone.

Furthermore, in the present disclosure, the racemic cis-2 - (2-chloropropenyl) - 3-hydroxypiperidine is chiral separated with chiral dibenzoyl tartaric acid to obtain optically active cis-2 - (2-chloropropenyl) - 3-hydroxypiperidine, which is used to prepare the optically active halofuginone. The basic nitrogen atom of cis-2 - (2-chloropropenyl) - 3-hydroxypiperidine can salt formation react with chiral dibenzoyl tartaric acid. After salt formation, cis-2 - (2-chloropropenyl) - 3-hydroxypiperidine with optical activity can be obtained by simple crystal resolution. The chiral dibenzoyl tartaric acid used in the synthesis method of the present disclosure has many advantages, such as having wide sources, low price, simple preparation process, fewer product impurities, no need for column chromatography purification, and can be used for large-scale production and preparation for halofuginone with high optical purity. The synthesis method of chiral synthesis and its intermediates of the present disclosure solves the technical defects in the current synthesis for halofuginone, with many advantages such as simple synthesis process, low cost, fewer by-products in the synthesis process, simple purification process, no need for column chromatography purification, high product yield, fewer impurity, high purity, and controllable product quality. It overcomes the disadvantages of the existing synthetic routes of optically active halofuginone or febrifugine, such as harsh reaction conditions, low yield, complex separation and purification, or the use of highly toxic and expensive reagents.

### Detailed Description

The synthesis method for halofuginone and halofuginone intermediate according to the present disclosure is further described below through specific embodiments.

In the present disclosure, the compounds shown in Formula 8, Formula 9, Formula 10, Formula 11, Formula 13 and Formula 1 are referred as racemic compound when they are not marked with (+) or (-), or marked with (±) . The stereoscopic structure in the Formula is relative configuration, not absolute configuration; the compounds in Formula 8, Formula 9, Formula 10, Formula 11, Formula 13 and Formula 1 are referred as chiral compounds with optical activity when marked with (+) or (-), and the stereostructure in the Formula is absolute configuration.

In one aspect, the present disclosure provides a synthesis method for the halofuginone intermediate having the structure as shown in Formula 9, comprises the following steps: 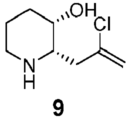
(a) alkylation reacting diethyl acetaminomalonate with 2,3-dichloropropene under the action of a base and a catalyst to form the compound of Formula 2;
(b) decarboxylation reacting the compound of Formula 2 in the presence of an acid catalyst to produce the compound of Formula 3;
(c) esterification reacting the compound of Formula 3 in the presence of an acid catalyst to produce the compound of Formula 4;
(d) nitrogen alkylation reacting the compound of Formula 4 with 4-halogenated butyrate under the action of a base and a catalyst, and then nitrogen protection reacting with amino protection reagent to produce the compound of Formula 5;
(e) Dieckmann condensation reacting the compound of Formula 5 under the action of the base to produce the compound of Formula 6;
(f) decarboxylation reacting the compound of Formula 6 in the presence of inorganic salt to produce the compound of Formula 7;
(g) reduction reacting reacting the compound of Formula 7 to produce the compound of Formula 8;
(h) nitrogen deprotection reacting the compound of Formula 8 to produce the compound of Formula 9;
   the reaction Formulas are as following: 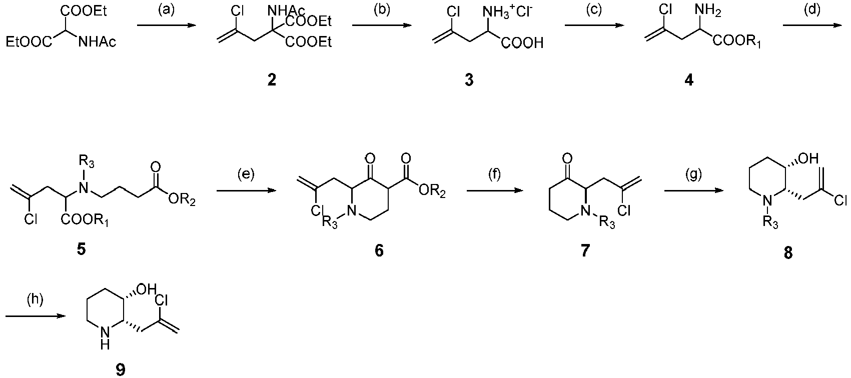
wherein,
R₁ is selected from: methyl, ethyl, propyl, isopropyl or tert -butyl, preferably methyl and ethyl;
R₂ is selected from: methyl, ethyl;
R₃ is selected from: methoxyformyl, ethoxyformyl, tert-butoxyformyl, benzyloxyformyl, trichloroethoxyformyl or benzyl, preferably benzyloxyformyl.

In some embodiments, the base in step (a) is at least one selected from potassium carbonate, cesium carbonate, sodium carbonate, sodium hydroxide, lithium hydroxide, potassium hydroxide, sodium methoxide, sodium ethoxide, sodium tert-butoxide, potassium tert-butoxide, sodium hydride, lithium hydride and potassium hydride.

In some embodiments, the catalyst in step (a) is the combination of quaternary ammonium salt and iodide; wherein the quaternary ammonium salt was any one selected from tetrabutylammonium bromide, tetraethylammonium bromide, tetrabutylammonium iodide and benzyl triethyl ammonium chloride; and the iodide is any one selected from sodium iodide, potassium iodide, and lithium iodide.

In some embodiments, the molar ratio of the quaternary ammonium salt to the iodide is 1:(2-6).

In some embodiments, the solvent used in the alkylation in step (a) is any one selected from acetonitrile, methanol, ethanol, N, N-Dimethylacetamide, N, N-dimethylformamide, dimethyl sulfoxide, tetrahydrofuran, 1,4-dioxane, toluene, dichloromethane and 1,2-dichloroethane.

In some embodiments, the reaction temperature of the alkylation in step (a) is 20°C-120°C.

In some embodiments, the molar ratio of Diethyl acetaminomalonate, 2,3-dichloropropene, catalyst, and base in step (a) is 1: (1- 2): (0.1- 0.5): (1- 3).

In some embodiments, the acid in step (b) is hydrogen chloride aqueous solution, and the concentration of the hydrogen chloride aqueous solution is 5-12mol/ L.

In some embodiments, the molar ratio of the compound of Formula 2 to hydrogen chloride is 1: (5-30).

In some embodiments, the acid in step (c) is any one selected from sulfuric acid, phosphoric acid, hydrochloric acid, and p-toluenesulfonic acid.

In some embodiments, the solvent for the esterification reaction in step (c) is at least one selected from ethanol, diethyl carbonate, dimethyl carbonate, methanol, propanol and benzyl alcohol.

In some embodiments, the reaction temperature of the esterification reaction in step (c) is 0°C-120 °C.

In some embodiments, the base used in step (d) is any one selected from potassium carbonate, potassium bicarbonate, cesium carbonate, sodium carbonate, sodium bicarbonate, sodium hydroxide, lithium hydroxide, potassium hydroxide, triethylamine, diisopropylethylamine, 1,8-diazabicyclo [5.4.0] undecan-7-ene.

In some embodiments, the 4-halobutyrate in step (d) is any one selected from 4-bromobutyrate, 4-chlorobutyrate, and 4-iodobutyrate.

In some embodiments, the catalyst in step (d) is quaternary ammonium salt or a combination of quaternary ammonium salt and iodide, wherein the quaternary ammonium salt is any one selected from tetrabutyl ammonium bromide, tetraethyl ammonium bromide, tetrabutyl ammonium iodide and benzyltriethylammonium chloride, and the iodide is any one selected from sodium iodide and potassium iodide.

In some embodiments, the solvent in the alkylation of nitrogen in step (d) is any one selected from acetonitrile, methanol, ethanol, N, N-Dimethylacetamide, N, N-dimethylformamide, dimethyl sulfoxide, tetrahydrofuran, 1,4-dioxane, toluene, dichloromethane and 1,2-dichloroethane.

In some embodiments, the reaction temperature of the alkylation of nitrogen in step (d) is 20°C-120°C.

In some embodiments, in step (d), the molar ratio of compound of Formula 4, 4-halobutyrate, base, and catalyst is 1:(1-1.5): (1-3): (0.01-0.2).

In some embodiments, the amine protection reagent in step (d) is any one selected from benzyl chloroformate, di-tert-butyl dicarbonate, methyl chloroformate, ethyl chloroformate, trichloroethyl chloroformate, benzyl bromide, and benzyl chloride.

In some embodiments, in step (d), the molar ratio of the amine protection reagent to the compound of Formula 4 is (0.8-2): 1.

In some embodiments, the reaction temperature of the nitrogen protection reaction in step (d) is 0°C -100°C.

In some embodiments, the 4-halobutyrate in step (d) is Ethyl 4-bromobutyrate.

In some embodiments, the amine protection reagent in step (d) is benzyl chloroformate, and the molar ratio of benzyl chloroformate to the compound of Formula 4 is (0.8~1.2):1, and the reaction temperature is 0°C~50°C.

In some embodiments, the base in step (e) is any one selected from sodium methoxide, sodium ethoxide, sodium tert-butoxide, potassium tert-butoxide, sodium hydride, lithium hydride, lithium diisopropylamide, sodium bis-(trimethylsilyl) amide, lithium bis-(trimethylsilyl) amide and potassium bis-(trimethylsilyl) amide.

In some embodiments, the solvent of Dieckmann condensation reaction in step (e) is any one or combination of two selected from tetrahydrofuran, toluene, xylene, methyl tert- butyl ether, methanol, and ethanol.

In some embodiments, in step (e), the molar ratio of base to the compound of Formula 5 is (1- 3):1.

In some embodiments, the reaction temperature of the Dieckmann condensation in step (e) is -20 °Cto 80°C.

In some embodiments, the inorganic salt in step (f) is any one selected from sodium chloride, lithium chloride, sodium bromide, and lithium bromide.

In some embodiments, the molar ratio of the inorganic salt to the compound of Formula 6 in step (f) is (1- 3):1.

In some embodiments, the reaction solvent of decarboxylation in step (f) is a combination of organic solvent and water, wherein the organic solvent is any one selected from dimethyl sulfoxide, sulfolane, N-methylpyrrolidone, N, N-dimethylacetamide, and N, N-dimethylformamide.

In some embodiments, the volume mass ratio of the organic solvent, water and the compound of Formula 6 in step (f) is (3-5):(0.1-1):1.

In some embodiments, the reaction temperature of decarboxylation reaction in step (f) is 100°C to 150 °C.

In some embodiments, the inorganic salt in step (f) is lithium chloride, and the reaction solvent for decarboxylation is the combination of N, N-dimethylformamide and water.

In some embodiments, the reducing agent in step (g) is any one selected from sodium borohydride, potassium borohydride, lithium borohydride, lithium aluminum hydride, sodium bis(2-methoxyethoxy) aluminumhydride, borane, sodium amalgam and lithium tri-tert-butoxyaluminum hydride; preferably sodium borohydride.

In some embodiments, the solvent of the reduction reaction in step (g) is ethanol.

In some embodiments, the molar ratio of the reducing agent to the compound of Formula 7 in step (g) is (1-2):1.

In some embodiments, the reduction reaction temperature in step (g) is 0°C-10 °C.

In some embodiments, R₃ is benzyloxyformyl, and the compound of Formula 8 undergoes nitrogen deprotection reaction in the presence of acid to produce the compound of Formula 9, wherein the acid is at least one selected from hydrochloric acid, hydrobromic acid and sulfuric acid; and the solvent for the deprotection reaction in step (h) is acetic acid, water or the combination of water and alcohol, and the alcohol is any one from methanol, ethanol and isopropanol.

In some embodiments, R₁ is ethyl; R₂ is ethyl; R₃ is benzyloxyformyl.

On the other hand, the present disclosure provides a synthesis method for optically active halofuginone, which is a synthesis method for cis-2- (2-chloropropenyl)- 3-hydroxypiperidine with optical activity, comprising the following steps of:
(1) In the first organic solvent, salt formation reacting the racemic cis-2-(2-chloropropenyl)-3-hydroxypiperidine with dibenzoyl tartaric acid or its derivatives to produce a precipitate, which is recrystallized to obtain a chiral complex salt;
(2) In the second organic solvent, the chiral complex salt is neutralized to alkalinity with an alkaline aqueous solution to obtain cis-2-(2-chloropropenyl)-3-hydroxypiperidine with optical activity;

The racemic cis-2 - (2-chloropropenyl) - 3-hydroxypiperidine has the structure shown in Formula (±)- 9; the optically active cis-2-(2-chloropropenyl)-3-hydroxypiperidine has the structure shown in Formula (+) - 9 or Formula (-) - 9; the dibenzoyl tartaric acid or its derivative has the structure shown in Formula 15 or Formula 16; the chiral complex salt has the structure shown in Formula 14 or Formula 17; the reaction equation is as follows: 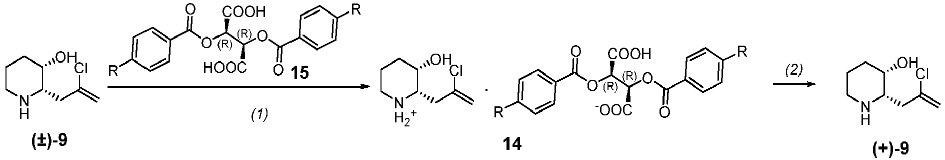 or 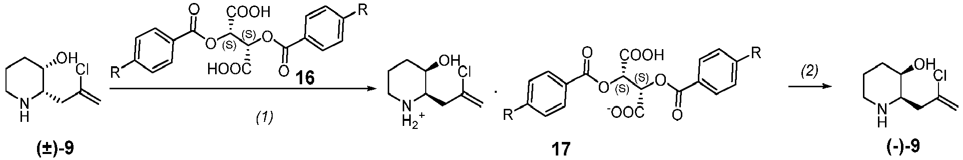 wherein each R is independently selected from: hydrogen or C₁-C₄ alkoxy.

In some embodiments, the dibenzoyl tartaric acid of Formula 15 or its derivative in step (1) is L- (-)- dibenzoyl tartaric acid or L- (-)-di-p-methoxybenzoyl tartaric acid, and the dibenzoyl tartaric acid of Formula 16 or its derivative is D-(+)-dibenzoyl tartaric acid or D-(+)-di-p-methoxybenzoyl tartaric acid.

In some embodiments, the molar ratio of racemic cis-2-(2-chloropropenyl)-3-hydroxypiperidine to dibenzoyl tartaric acid or its derivatives in step (1) is 1:(1-2).

In some embodiments, the recrystallization is carried out in a mixed solvent of a third organic solvent and water with a volume ratio of (1-10):1; the third organic solvent is any one or more selected from ethanol, methanol, isopropanol, acetonitrile, 1,4-dioxane, and acetone.

In some embodiments, the recrystallization is carried out in a mixed solvent of a third organic solvent and water with a volume ratio of (3-5):1, wherein the third organic solvent is acetonitrile.

In some embodiments, the first organic solvent in step (1) is any one or more selected from ethanol, methanol, isopropanol, acetonitrile, dichloromethane, 1,4-dioxane, tetrahydrofuran, toluene, acetone, and ethyl acetate.

In some embodiments, the second organic solvent described in step (2) is any one or more selected from ethyl acetate, dichloromethane, and trichloromethane.

In some embodiments, the temperature of the salt formation reaction is 0°C-100°C.

In some embodiments, the temperature of the salt formation reaction is 20°C- 40°C.

In some embodiments, the temperature of recrystallization is 0°C-30°C.

In some embodiments, the temperature of recrystallization is 15°C-28°C.

In some embodiments, the alkaline aqueous solution in step (2) is any one selected from sodium hydroxide aqueous solution, potassium hydroxide aqueous solution, lithium hydroxide aqueous solution, potassium carbonate aqueous solution, and sodium carbonate aqueous solution, and the neutralization to alkalinity is to pH 8-14.

In some embodiments, the synthesis method for racemic cis-2-(2-chloropropenyl)- 3-hydroxypiperidine with optical activity comprises the following steps:
(a) alkylation reacting Diethyl acetaminomalonate with 2,3-dichloropropene under the action of a base and a catalyst to form the compound of Formula 2;
(b) decarboxylation reacting the compound of Formula 2 in the presence of an acid catalyst to produce the compound of Formula 3;
(c) esterification reacting the compound of Formula 3 in the presence of an acid catalyst to produce the compound of Formula 4;
(d) nitrogen alkylation reacting the compound of Formula 4 with 4-halogenated butyrate under the action of a base and a catalyst, then nitrogen protection reacting with an amino protection reagent to produce the compound of Formula 5;
(e) Dieckmann condensation reacting the compound of Formula 5 under the action of a base to produce the compound of Formula 6;
(f) decarboxylation reacting the compound of Formula 6 in the presence of the inorganic salt to produce the compound of Formula 7;
(g) reduction reacting the compound of Formula 7 to produce the compound of Formula 8;
(h) nitrogen deprotection reacting the compound of Formula 8 to produce the compound of Formula 9; the reaction formulas are as follows:

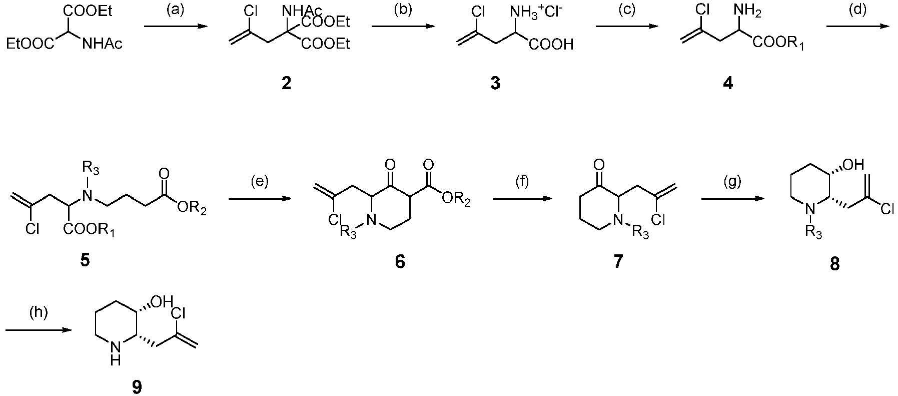

R₁ is selected from: methyl, ethyl, propyl, isopropyl or tert-butyl; preferably, methyl and ethyl;
R₂ is selected from: methyl, ethyl;
R₃ is selected from: methoxyformyl, ethoxyformyl, tert-butoxyformyl, benzyloxyformyl, trichloroethoxyformyl or benzyl, preferably, benzyloxyformyl.

In the third aspect, the present disclosure also provides a synthesis method for racemic halofuginone with the structure shown in Formula 1, comprising the following steps: 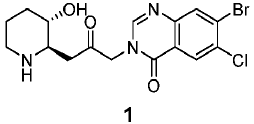
(i) reacting the compound of Formula 9 with the amino protection reagent under the action of alkali to produce a compound of Formula 10;
(j) reacting the compound of Formula 10 with olefin halogenation reagent and water, to produce a compound of Formula 11;
(k) reacting the compound of Formula 11 with the compound of Formula 12 under the action of base, then removing the 9-fluorenylmethoxyformyl protecting group on piperidine ring nitrogen, to produce a compound of Formula 13;
(l) isomerization reacting the compound of Formula 13 to produce a compound of Formula 1. 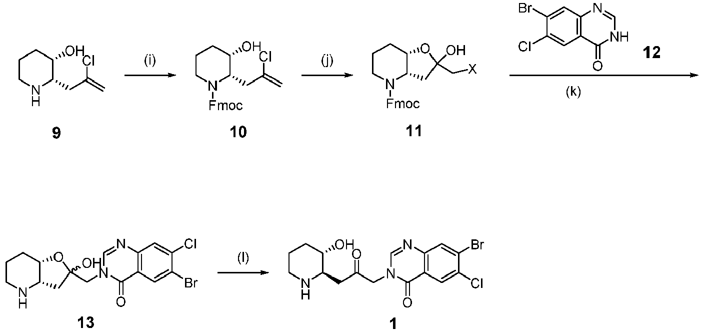
wherein, X is chlorine, bromine or iodine, preferably bromine.

In some embodiments, the base in step (i) is any one selected from sodium carbonate, sodium bicarbonate, potassium carbonate, and potassium bicarbonate.

In some embodiments, the amino protection reagent described in step (i) is any one selected from 9-fluorenylmethyl chloroformate, 9-fluorenylmethyl-1-benzotriazolyl carbonate, and 9-fluorenylmethyl-n-succinimide carbonate.

In some embodiments, the solvent in step (i) is a combination of organic solvent and water, wherein the organic solvent is any one selected from 1,4-dioxane and tetrahydrofuran.

In some embodiments, the molar ratio of the base, the amino protection reagent and the compound of Formula 9 in step (i) is (1-5):(1-2):1.

In some embodiments, the reaction temperature in step (i) is 0-20 °C.

In some embodiments, the olefin halogenation reagent in step (j) is any one selected from N-bromosuccinimide, N-chlorosuccinimide, n-iodobutanimide, trichloroisocyanuric acid, 1,3-dichloro-5,5-dimethylhydantoin, and 1,3-dibromo-5,5-dimethylhydantoin.

In some embodiments, the solvent in step (j) is any one selected from acetonitrile, tetrahydrofuran, and 1,4-dioxane.

In some embodiments, the molar ratio of the alkene halogenation reagent in step (j) to the compound of Formula 10 is (0.9-1.2):1.

In some embodiments, the reaction temperature in step (j) is - 10 °Cto 35 °C.

In some embodiments, the olefin halogenation reagent in step (j) is N-bromosuccinimide; wherein the molar ratio of the olefin halogenation reagent to the compound of formula 10 is (0.9-1):1; and the reaction solvent is acetonitrile; and the reaction temperature is -10°C to 10°C.

In some embodiments, the base in step (k) is any one selected from potassium carbonate, sodium carbonate, sodium hydroxide, potassium hydroxide, lithium hydroxide, cesium hydroxide, cesium carbonate, sodium methoxide, sodium ethoxide, sodium tert-butoxide potassium tert-butoxide, sodium hydride, lithium hydride, lithium diisopropylamide, sodium bis-(trimethylsilyl) amide, lithium bis-(trimethylsilyl) amide, and potassium bis-(trimethylsilyl) amide.

In some embodiments, the solvent in step (k) is any one selected from acetonitrile, methanol, ethanol, N, N-dimethylacetamide, N, N-dimethylformamide, dimethyl sulfoxide, tetrahydrofuran, 1,4-dioxane, toluene, dichloromethane, and 1,2-dichloroethane.

In some embodiments, the molar ratio of the compound of Formula 12 in step (k) and the compound of Formula 11, and the base is 1:(1-2): (0.8-1.2).

In some embodiments, the reaction temperature in step (k) is -10°C to 25°C.

In some embodiments, the base used in step (k) is potassium hydroxide. The reaction solvent is N, N-dimethylacetamide. The molar ratio of the compound of Formula 12 and the base, the compound of Formula 11 is 1: (1~1.1):(0.8~1.2), and the reaction temperature in step (k) is - 10°C to 25°C.

In some embodiments, the reaction for removing the 9-fluorenylmethoxyformyl protecting group from piperidine ring in step (k), is carried out under the action of secondary organic amine or tertiary organic amine, preferably diethylamine.

In some embodiments, the reaction temperature for removing the 9-fluorenylmethoxyformyl protecting group from the piperidine ring in step (k) is - 10 °Cto 25 °C.

In some embodiments, the solvent for the isomerization reaction in steps (1) is any one or a combination selected from water, ethanol, methanol, n-butanol, n-propanol, tert- butanol, N, N-dimethylformamide, tetrahydrofuran and 1,4-dioxane.

In some embodiments, the reaction temperature of the isomerization reaction in step (1) is 50-80 °C.

In some embodiments, the synthesis method of the said halofuginone comprises the following steps:
(a) alkylation reacting diethyl acetaminomalonate with 2,3-dichloropropene under the action of a base and a catalyst to form the compound of Formula 2;
(b) decarboxylation reacting the compound of Formula 2 in the presence of an acid catalyst to produce the compound of Formula 3;
(c) esterification reacting the compound of Formula 3 in the presence of an acid catalyst to produce the compound of Formula 4;
(d) nitrogen alkylation reacting the compound of Formula 4 with 4-halogenated butyrate under the action of a base and a catalyst, and then nitrogen protection reacting with amino protection reagent to produce the compound of Formula 5;
(e) Dieckmann condensation reacting the compound of Formula 5 under the action of the base to produce the compound of Formula 6;
(f) decarboxylation reacting the compound of Formula 6 in the presence of inorganic salt to produce the compound of Formula 7;
(g) reduction reacting the compound of Formula 7 under the action of a reducing agent to produce the compound of Formula 8;
(h) nitrogen deprotection reacting the compound of Formula 8 to produce the compound of Formula 9;
the reaction formulas are as follows: 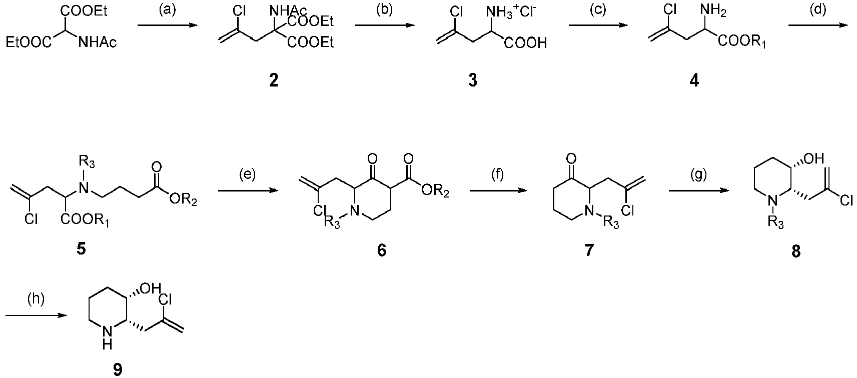 wherein,
R₁ is selected from: methyl, ethyl, propyl, isopropyl or tert -butyl; preferably, methyl and ethyl;
R₂ is selected from: methyl, ethyl;
R₃ is selected from: methoxyformyl, ethoxyformyl, tert-butoxyformyl, benzyloxyformyl, trichloroethoxyformyl or benzyl, preferably, benzyloxyformyl.

In the fourth aspect, the present disclosure also provides a synthesis method for halofuginone salt, included the following steps: synthesizing halofuginone by the synthesis method of halofuginone descried above, then reacting the halofuginone with acid to obtain the halofuginone salt.

In some embodiments, the acid is hydrobromic acid, hydrochloric acid or lactic acid.

Understandably, in the above method for synthesizing halofuginone and halofuginone salt, the reaction products obtained from any step through step (a) to (g) and (1) to (6) can be used as raw materials to directly carry out subsequent reactions to synthesize halofuginone salt. For example, a compound of Formula 9 can be used as a raw material to synthesize halofuginone and halofuginone salts through steps (i) to (l) as described above, or a compound of Formula 3 can be used as a raw material to synthesize halofuginone and halofuginone salt through steps (c) to (l) as described above.

In the fifth aspect, the present disclosure also provides a synthesis method for halofuginone with optical activity, comprising the following steps:
(3) reacting the optically active cis-2 - (2-chloropropenyl) - 3-hydroxypiperidin with an amino protecting agent in the presence of a base to form optically active compounds of Formula (+) - 10 or Formula (-) - 10;
(4) reacting the optically active compound of Formula (+) - 10 or Formula (-) - 10 with olefin bromination reagent and water to form optically active compound of Formula (+) - 11 or Formula (-) - 11;
(5) reacting the optically active compound of Formula (+) - 11 or Formula (-) - 11 with the compound of Formula F2 under the action of a base, and then the 9-fluorenylmethoxyformyl protecting group on the piperidine ring nitrogen is removed to form the optically active compound of Formula (+) - 13 or Formula (-) - 13;
(6) The optically active compound of Formula (+) - 13 or Formula (-) - 13 is isomerized to produce the optically active halofuginone.
the optically active halofuginone has the structure shown in Formula (+) - 1 or Formula (-) - 1, and the reaction equation is as follows: 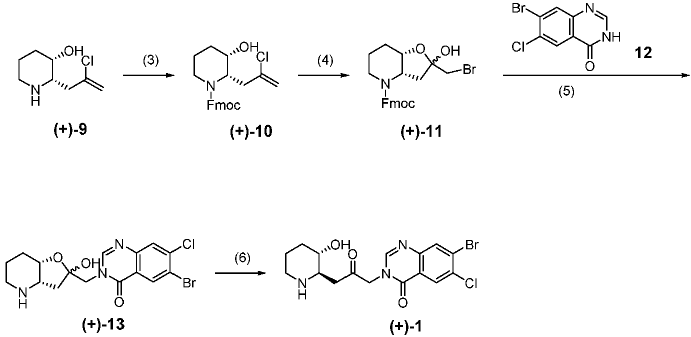 or

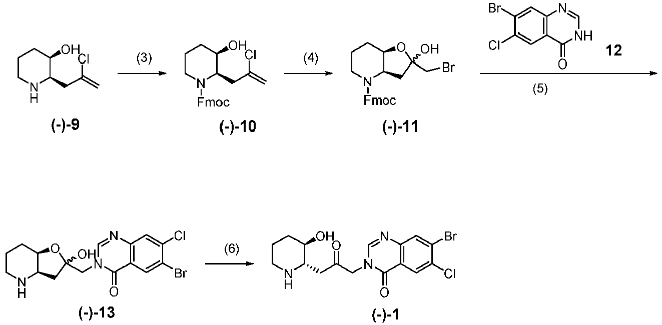

In some embodiments, the alkali in step (3) is any one selected from sodium carbonate, sodium bicarbonate, potassium carbonate, and potassium bicarbonate.

In some embodiments, the amino protection reagent described in step (3) is any one selected from 9-fluorenylmethyl chloroformate, 9-fluorenylmethyl-1-benzotriazolyl carbonate, and 9-fluorenylmethyl-n-succinimide carbonate.

In some embodiments, the solvent in step (3) is a combination of organic solvent and water, and the organic solvent is either 1,4-dioxane or tetrahydrofuran.

In some embodiments, the molar ratio of the base, the amino protection reagent and cis-2 - (2-chloropropenyl) - 3-hydroxypiperidin in step (3) is (1-5):(1-2):1.

In some embodiments, the reaction temperature in step (3) is 0°C-20 °C.

In some embodiments, the olefin halogenation reagent in step (4) is any one selected from N-bromosuccinimide and 1,3-dibromo-5,5-dimethylhydantoin.

In some embodiments, the solvent in step (4) is any one selected from acetonitrile, tetrahydrofuran and 1,4-dioxane.

In some embodiments, the molar ratio of the alkene halogenation reagent in step (4) to the compound of Formula (+)-10 or Formula (-) -10 is (0.9-1.2):1.

In some embodiments, the reaction temperature in step (4) is - 10 °Cto 35 °C.

In some embodiments, the reaction temperature is -10°C to 10°C.

In some embodiments, the base in step (5) is any one selected from potassium carbonate, sodium carbonate, sodium hydroxide, potassium hydroxide, lithium hydroxide, cesium hydroxide, cesium carbonate, sodium methoxide, sodium ethoxide, sodium tert-butoxide, potassium tert-butoxide, sodium hydride, lithium hydride, diisopropyl amino lithium, sodium bis-(trimethylsilyl) amide, lithium bis-(trimethylsilyl) amide, and potassium. bis-(trimethylsilyl) amide

In some embodiments, the solvent in step (5) is any one selected from acetonitrile, methanol, ethanol, N, N-Dimethylacetamide, N, N-dimethylformamide, dimethyl sulfoxide, tetrahydrofuran, 1,4-dioxane, toluene, dichloromethane, and 1,2-dichloroethane.

In some embodiments, the molar ratio of the compound of Formula (+) -11 or Formula (-) - 11 in step (5), the compound of Formula 12, and the base is (0.8-1.2) : 1 : (1-2).

In some embodiments, the reaction temperature in step (5) is -10°C to 25 °C.

In some embodiments, the reaction for removing the 9-fluorenylmethoxyformyl protecting group from piperidine ring in step (5) is carried out under the action of secondary organic amine or tert-iary organic amine.

In some embodiments, the reaction temperature for removing the 9-fluorenylmethoxyformyl protecting group from the piperidine ring in step (5) is - 10°C to 25 °C.

In some embodiments, the secondary organic amine or the tertiary organic amine is diethylamine.

In some embodiments, the solvent for the isomerization reaction in steps (6) is any one or a combination of two selected from water, ethanol, methanol, n-butanol, n-propanol, tert- butanol, N, N-dimethylformamide, tetrahydrofuran, and 1,4-dioxane.

In some embodiments, the reaction temperature of the isomerization reaction in step (6) is 50-80 °C.

In some embodiments, the synthesis method for the optically active halofuginone also includes the step of preparing cis-2- (2-chloropropenyl) - 3-hydroxypiperidine with optical activity, comprising the following steps:
(1) In the first organic solvent, salt formation reacting the racemic cis-2-(2-chloropropenyl)-3-hydroxypiperidine with dibenzoyl tartaric acid or its derivatives to produce a precipitate, which is recrystallized to obtain a chiral complex salt;
(2) In the second organic solvent, the chiral complex salt is neutralized to alkalinity with an alkaline aqueous solution to obtain cis-2-(2-chloropropenyl)-3-hydroxypiperidine with optical activity;

The racemic cis-2 - (2-chloropropenyl) - 3-hydroxypiperidine has the structure shown in Formula (±)- 9; the optically active cis-2-(2-chloropropenyl)-3-hydroxypiperidine has the structure shown in Formula (+) - 9 or Formula (-) - 9; the dibenzoyl tartaric acid or its derivative has the structure shown in Formula 15 or Formula 16; the chiral complex salt has the structure shown in Formula 14 or Formula 17; the reaction equation is as follows: 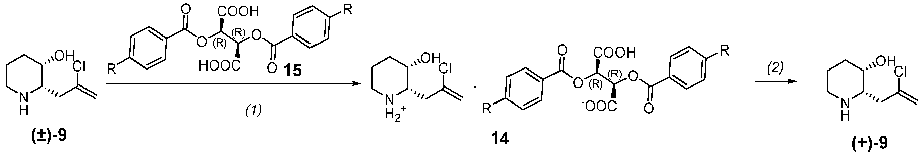 or, 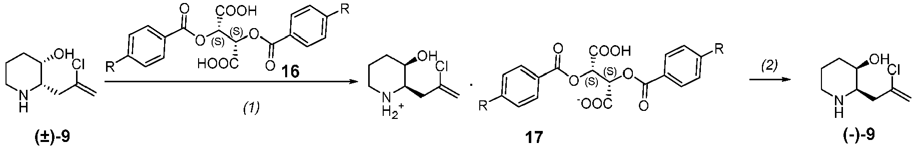 wherein each R is independently selected from: hydrogen or C₁-C₄ alkoxy.

In some embodiments, the synthesis method for racemic cis-2 - (2-chloropropenyl) - 3-hydroxypiperidine with optical activity comprises the following steps:
(a) alkylation reacting Diethyl acetaminomalonate with 2,3-dichloropropene under the action of base and catalyst to form the compound of Formula 2;
(b) decarboxylation reacting the compound of Formula 2 in the presence of an acid catalyst to produce the compound of Formula 3;
(c) esterification reacting the compound of Formula 3 in the presence of an acid catalyst to produce the compound of Formula 4;
(d) nitrogen alkylation reacting the compound of Formula 4 with 4-halogenated butyrate under the action of abase and a catalyst, then nitrogen protection reacting with an amino protection reagent to produce the compound of Formula 5;
(e) Dieckmann condensation reacting the compound of Formula 5 under the action of a base to produce the compound of Formula 6.
(f) decarboxylation reacting the compound of Formula 6 in the presence of the inorganic salt to produce the compound of Formula 7.
(g) reduction reacting the compound of Formula 7 to produce the compound of Formula 8;
(h) nitrogen deprotection reacting the compound of Formula 8 to produce the compound of Formula 9.
the reaction formulas are as follows: 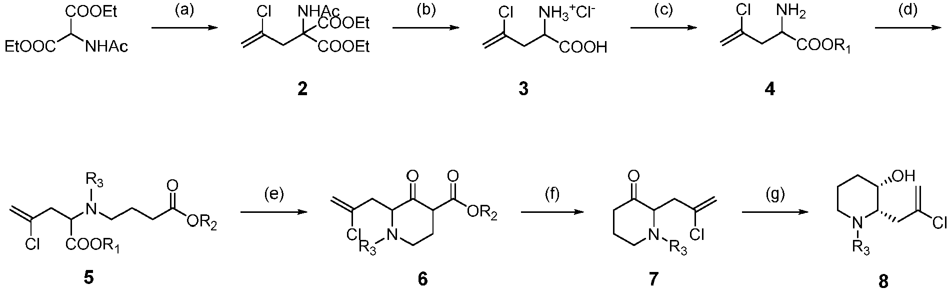 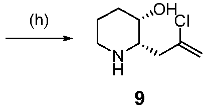 wherein,
R₁ is selected from: methyl, ethyl, propyl, isopropyl or tert-butyl; preferably, methyl and ethyl;
R₂ is selected from: methyl, ethyl;
R₃ is selected from: methoxyformyl, ethoxyformyl, tert--butoxyformyl, benzyloxyformyl, trichloroethoxyformyl or benzyl, preferably, benzyloxyformyl.

In the sixth aspect, the present disclosure also provides a synthesis method for optically active halofuginone salt, comprising the following steps: synthesizing optically active halofuginone by the synthesis method for halofuginone descried above, then reacting the halofuginone with acid to obtain the optically active halofuginone salt.

In some embodiments, the acid is hydrobromic acid, hydrochloric acid or lactic acid.

Understandably, in the above method for synthesizing optically active halofuginone and its salt, the reaction products obtained from any step through step (a) to (g) and (1) to (6) can be used as raw materials to directly carry out subsequent reactions to synthesize optically active halofuginone. For example, the racemic cis-2 - (2-chloropropenyl) - 3-hydroxypiperidine can be used as the raw material to synthesize the optically active halofuginone through step (1) to (6) as described above, or the compound of Formula 3 can be used as the raw material to synthesize the optically active halofuginone through step (c) to (g) and (1) to (6) as described above.

### Embodiments

### Embodiment 1 Synthesis of racemic halofuginone

### Preparation of intermediate compound of Formula 4 (R₁=Et)

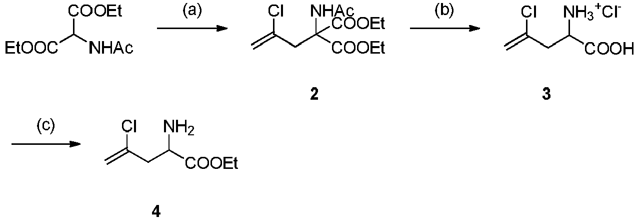

Step (a): At room temperature, diethyl acetylaminomalonic acid (5.00kg, 23.02mol), anhydrous potassium carbonate (6.35kg, 46.04mol), potassium iodide (0.76kg, 4.6mol), tetrabutylammonium bromide (0.37kg, 1.15mol) and acetonitrile (25L) were added into a 50L reaction kettle. After being stirred for 20 minutes, 2,3-dichloropropene (3.07kg, 27.62mol) was added. The reaction was carried out at 85-90 °C and monitored by HPLC. After the end of the reaction, the reaction mixture was cooled to a temperature within 25 °C, then added dropwise with diluted hydrochloric acid to neutralize the reaction mixture to pH 7-7.5. The reaction solution was left to separation, then the organic layer was concentrated under reduced pressure at 50 °C. The concentrated residue was added with ethanol-water (1:10, 20L) and stirred for 1 hour for crystallization. After vacuum filtration, the filter cake was washed with water to obtain compound of Formula 2, which was a yellow solid (wet weight 10.50kg), which was not dried and directly put into the next reaction.

¹H NMR (500 MHz, Chloroform-*d*) δ 5.28 (d, *J =* 1.2 Hz, 1H), 5.17 (d, *J =* 1.1 Hz, 1H), 4.26 (qd, *J* = 7.1, 2.4 Hz, 4H), 3.47 (s, 2H), 2.03 (s, 3H), 1.55 - 1.51 (m, 1H), 1.27 (t, *J* = 7.1 Hz, 6H). ¹³C NMR (126 MHz, Chloroform-*d*) δ 169.3, 167.3, 136.5, 117.8, 65.2, 63.0, 41.6, 23.0, 14.0.

HRMS (m/z): calc. for C₁₂H₁₉ClNO₅ [M+H]⁺= 292.0952 ; found, 292.0954

Step (b): At room temperature, the compound of Formula 2 (wet weight 10.5kg, 23.02mol) and hydrochloric acid solution (6mol / L, 57.5l, 345.27mol) were added into a 100L reaction kettle, stirred and heated to 100 °C for reflux reaction, and the reaction was monitored by TLC. After the end of the reaction, activated carbon (650g) was added to the reaction kettle and cooled to not to exceed 50 °C. After vacuum filtration, the filter cake was washed with water, then the filtrate was combined and concentrated under reduced pressure at 80-85 °C to remove the solvent to obtain compound of Formula 3, which was a light-yellow solid (4.05kg). The product was directly put into the next reaction without further purification.

Step (c): At room temperature, compound of Formula 3 (4.05kg, 21.77mol), diethyl carbonate (12L) and anhydrous ethanol (4L) were added into a 50L reaction kettle and stirred. Concentrated sulfuric acid (1.13kg, 11.51mol) was added dropwise and completed within 20-30 minutes. Then, heated to 85-95 °C for reflux reaction, and the reaction was monitored by HPLC. After the end of the reaction, the reaction solution was cooled to 50-60 °C and concentrated under reduced pressure, and the remaining liquid was diluted with water and cooled to not to exceed 10 °C. 30% sodium hydroxide was added dropwise to adjust the solution to pH 8-9, then moved to room temperature for extraction using dichloromethane (10L × 3). After extraction, the organic layers were combined, added with anhydrous sodium sulfate and dried. After vacuum filtration, the filtrate was concentrated at 40 °C to obtain compound of Formula 4 which is a light brown oil (3.07 kg, The total yield of 3 steps is 75%).

¹H NMR (500 MHz, Chloroform-*d*) δ 5.30 (d, *J =* 1.3 Hz, 1H), 5.26 (q, *J =* 1.1 Hz, 1H), 4.20 (q, *J=* 7.1 Hz, 2H), 3.78 (dd, *J* = 8.6, 4.8 Hz, 1H), 2.81 (ddd, *J* = 14.2, 4.8, 1.1 Hz, 1H), 2.56 (dd, *J =* 14.2, 8.6 Hz, 1H), 1.28 (t, *J=* 7.1 Hz, 3H).

¹³C NMR (126 MHz, Chloroform-d) δ 174.5, 138.5, 115.9, 61.4, 52.3, 44.6, 14.3.

HRMS (m/z): calc. for C₇H₁₃ClNO₂ [M+H]⁺= 178.0635 ; found, 178.0628.

Note: in step (a), the product was wet without being dried, so the yield exceeded the theoretical value. Therefore, according to the 100% yield, the mole number of the compound of Formula 2 was 23.02 mol.

### Preparation of intermediate compound of Formula 6 (R₁=Et; R₂=Et; R₃=Cbz)

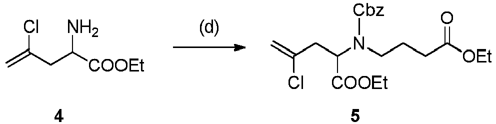

Step (d): At room temperature, compound of Formula 4 (3.07kg, 17.26mol), anhydrous sodium carbonate (5.49kg, 51.79mol), tetrabutylammonium iodide (0.64kg, 1.73mol) and toluene (9L) were added into a 50L reaction kettle, and toluene (6L) solution of ethyl 4-bromobutyrate (3.37kg, 17.26mol) was added after stirring for 20 minutes. The reaction was stirred at 75-80 °C and monitored by HPLC. After the end of the reaction, the temperature was cooled to 20-25 °C. Water (9L) was added and stirred for 10-15 minutes, then benzyl chloroformate (2.94kg, 17.26mol) was added dropwise and completed within about 2-3 hours. The reaction was continuously stirred at 20-25 °C and monitored by TLC. After the reaction, water (10L) and toluene (10L) were added and stirred for 0.5h to separate the solution. The organic layer was successively washed with 5% NaOH (15L), water (20L), 5% hydrochloric acid (15L) and water (20L). The organic layer was added with activated carbon (250.0g) and stirred at room temperature for 1 hour. After vacuum filtration, the filtrate was concentrated under reduced pressure at 60 °C to obtain compound 5 which was a brown oil (8.10 kg). The product was directly put into the next reaction without further purification.

¹H NMR (500 MHz, Chloroform-*d*) δ 7.39 - 7.27 (m, 5H), 5.19 (d, *J* = 1.3 Hz, 1H), 5.17 - 5.07 (m, 2.6H), 5.02 (s, 0.4H), 4.27 - 3.89 (m, 5H), 3.68 - 3.56 (m, 1H), 3.23 - 3.10 (m, 1.6H), 3.02 - 2.90 (m, 1.4H), 2.47 - 2.26 (m, 2H), 1.99 -1.90 (m, 2H), 1.27 - 1.17 (m, 4.8H), 1.13 (t, *J =* 7.2 Hz, 1.2H).

¹³C NMR (126 MHz, Chloroform-*d*) δ 173.3, 173.2, 170.3, 170.2, 155.6, 138.8, 138.4, 136.7, 136.2, 128.7, 128.6, 128.4, 128.2, 127.9, 116.3, 116.0, 67.6, 67.4, 61.7, 60.5, 59.6, 58.7, 49.2, 48.9, 40.5, 39.4, 31.6, 31.4, 24.2, 23.7, 14.4, 14.14, 14.07.

HRMS (m/z): calc. for C₂₁H₂₉ClNO₆ [M+H]⁺= 426.1683 ; found, 426.1678

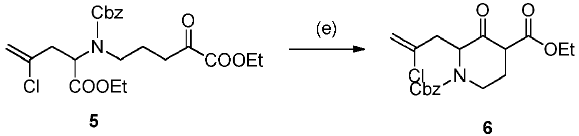

Step (e): At room temperature and under the protection of nitrogen, sodium tert-butoxide (3.31kg, 34.53mol) and anhydrous tetrahydrofuran (38L) were added into a 100L reaction kettle and stirred, then cooled to below -5 °C. The compound of Formula 5 (8.10kg, 17.26mol) was dissolved in tetrahydrofuran (15L) and then added dropwise into the reaction kettle. The temperature during the dripping process was controlled not to exceed 0 °C and complete dripping within 4-5 hours. Continued stirring at 0°C-5 °C after the dripping process and the reaction was monitored by HPLC. After the end of the reaction, dilute hydrochloric acid was added to the kettle to adjust the solution to pH5-6, then ethyl acetate(10L) was added, and stirred at room temperature to divide the solution. The organic layer was washed with saturated sodium chloride (20L × 2), and the aqueous layer is extracted once with ethyl acetate (10L). Then the organic layer was combined, and activated carbon (500g) was added and stirred for 1 hour at room temperature. After vacuum filtration, the filtrate was concentrated under reduced pressure to obtain compound of Formula 6 which is a light brown oil (5.44kg, yield of two steps is 83%).

¹H NMR (500 MHz, Chloroform-*d*) δ 12.23 (s, 1H), 7.41 - 7.28 (m, 5H), 5.27 - 5.05 (m, 4H), 5.01 (s, 0.4H), 4.94 - 4.82 (m, 0.6H), 4.30 (dd, *J* = 13.6, 5.7 Hz, 0.6H), 4.23 (q, *J* = 7.1 Hz, 2H), 4.19 - 4.12 (m, 0.4H), 3.09 - 2.66 (m, 3H), 2.46 - 2.23 (m, 2H), 1.30 (t, *J =* 7.1 Hz, 3H).

¹³C NMR (126 MHz, Chloroform-*d*) δ 172.0, 171.9, 168.8, 168.2, 155.2, 155.1, 138.4, 138.3, 136.7, 136.3, 128.5, 128.2, 128.0, 115.8, 115.6, 97.7, 97.4, 67.7, 67.5, 61.0, 52.7, 52.5, 41.5, 40.7, 38.2, 37.3, 22.8, 22.3, 14.3.

HRMS (m/z): calc. for C₁₉H₂₃ClNO₅ [M+H]⁺= 380.1265 ; found, 380.1266

Note: in step (d), the yield exceeded the theoretical value. Therefore, according to the 100% yield, the mole number of the compound of Formula 5 was 17.26 mol.

### Preparation of intermediate compound of Formula 7 (Rs=Cbz)

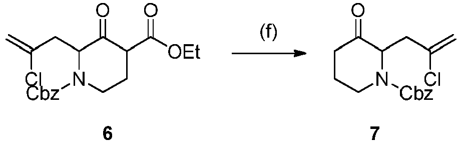

Step (f): Compound of Formula 6 (5.44kg, 14.33mol), DMF (16.3L), water (2.7L) and lithium chloride (0.61kg, 14.33mol) were added into a 50L reaction kettle at room temperature and stirred. The reaction mixture was stirred and heated to 120 °C, and the reaction was monitored by HPLC. After the end of the reaction, the reaction mixture was cooled to 20-25 °C. Water (25L) and methyl tert-butyl ether (30L) were added, stirred and separated. The organic layer was washed with water (25L × 2) and separated, then the generated aqueous layer was extracted with methyl tert-butyl ether (25L). By combining the organic layers and concentrating under reduced pressure at 45 °C, compound of Formula 7 (4.19 kg, yield 95%) was obtained, which was a brown oil.

¹H NMR (500 MHz, Chloroform-d) δ 7.40 - 7.29 (m, 5H), 5.32 - 5.03 (m, 4H), 4.86 (s, 1H), 4.34 - 3.98 (m, 1H), 3.23 (s, 1H), 2.94 - 2.59 (m, 2H), 2.59 - 2.39 (m, 2H), 2.05 (s, 1H), 2.01 - 1.92 (m, 1H).

¹³C NMR (126 MHz, Chloroform-d) δ 206.7, 155.5, 128.6, 128.3, 116.1, 67.8, 61.8, 37.1, 22.5.

HRMS (m/z): calc. for C₁₆H₁₉ClNO₃ [M+H]⁺= 308.1053 ; found, 308.1057

### Preparation of intermediate compound of Formula 8 (Rs=Cbz)

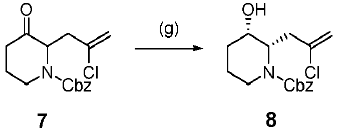

Step (g): Anhydrous ethanol (18L) and sodium borohydride (0.51kg, 13.61mol) were added into a 50L reaction kettle at room temperature and stirred, then cooled to 5-10 °C. Compound of Formula 7 (4.19kg, 13.61mol) was dissolved in anhydrous ethanol (9L) and added dropwise into the reactor under the temperature controlled not to exceed 10 °C. Keep stirring at 5- 10°C. The reaction was monitored by HPLC. After the end of the reaction, water (20L) was added dropwise into the kettle. After the dripping process, methyl tert-butyl ether (25L) was added, stirred and separated. The organic layer was washed sequentially with 10% NaOH (10L), 5% hydrochloric acid (10L×2) and Water (10L). The generated aqueous layer was extracted with methyl tert-butyl ether (25L). The organic layers were combined, and added with activated carbon (500g) and stirred at room temperature for 1 hour. After filtration, the filtrate was concentrated under reduced pressure at 45 °C to obtain the compound of Formula 8 (3.50kg, yield 83%), which was a brown oil.

¹H NMR (500 MHz, Chloroform-*d*) δ 7.42 - 7.27 (m, 5H), 5.21 - 5.06 (m, 4H), 4.76 (s, 1H), 4.05 (d, *J =* 14.0 Hz, 1H), 3.92 - 3.79 (m, 1H), 2.84 - 2.60 (m, 3H), 1.87 - 1.76 (m, 1H), 1.76 - 1.65 (m, 1H), 1.58 - 1.42 (m, 2H).

¹³C NMR (126 MHz, Chloroform-*d*) δ 155.8, 139.8, 136.7, 128.5, 128.2, 128.1, 114.5, 68.7, 67.5, 54.2, 37.9, 33.5, 27.9, 24.3.

HRMS (m/z): calc. for C₁₆H₂₁ClNO₃ [M+H]⁺= 310.1210 ; found, 310.1208

### Preparation of intermediate compound of Formula 9

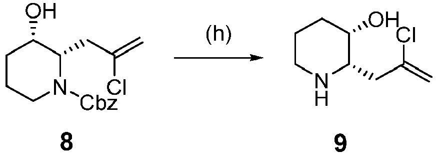

Step (h): Compound of Formula 8 (3.55kg, 12.36mol), hydrochloric acid (6mol / L, 18.83!, 112.98mol) and ethanol (20L) were added into a 50L reaction kettle at room temperature, stirred and heated for reflux reaction and the reaction was monitored by HPLC. After the reaction was completed, the reaction solution was cooled to 50-55 °C and concentrated under reduced pressure to remove ethanol. The remaining liquid was added with methyl tert- butyl ether (20L × 2), stirred and separated. The aqueous layer was adjusted to pH > 11 with 40% sodium hydroxide, and then added ethyl acetate (20L × 2) for extraction. The organic layers were combined, and added with anhydrous magnesium sulfate and dried. After filtration, the filtrate was concentrated under reduced pressure to obtain the crude product. Acetonitrile (5L) was added to the crude product, stirred to dissolve at 70 °C, and then the product was crystallized at room temperature. After filtration and vacuum drying, compound of Formula 9 (1.01 kg, yield 50.7%) was obtained, which was a nearly white solid.

¹H NMR (500 MHz, Chloroform-*d*) δ 5.25 (d, *J =* 1.1 Hz, 1H), 5.23 (t, *J =* 1.0 Hz, 1H), 3.65 (S, 1H), 3.03 (ddt, *J* = 11.5, 4.3, 2.0 Hz, 1H), 2.88 (ddd, *J* = 7.5, 6.3, 1.4 Hz, 1H), 2.65 (td, *J* = 11.9, 2.9 Hz, 1H), 2.49 (d, *J* = 6.8 Hz, 2H)., 1.91 (dtt, *J* = 13.4, 4.0, 2.0 Hz, 1H), 1.73 (qt, *J=* 13.1, 4.3 Hz, 1H), 1.54 (tdd, *J* = 13.3, 4.7, 2.5 Hz, 1H), 1.45 (ddq, *J* = 12.9, 4.9, 2.6 Hz, 1H).

¹³C NMR (126 MHz, Chloroform-*d*) δ 139.8, 115.2, 67.1, 57.7, 47.2, 43.0, 32.2, 20.4.

HRMS (m/z): calc. for C₈H₁₅ClNO [M+H]⁺= 176.0842 ; found, 176.0837

### Preparation of intermediate compound of Formula 13

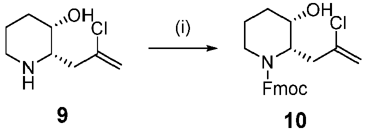

Step (i): Compound of Formula 9 (1kg, 5.69mol), 1,4-dioxane (5L), water (5L), sodium carbonate (0.91kg, 8.54mol) were added into a 50L reaction kettle at room temperature, stirred and cooled to 5°C-10 °C. 9-fluorenylmethyl chloroformate (1.47kg, 5.69mol) was dissolved in 1,4-dioxane (2L) and then added dropwise into the reaction kettle, with the temperature controlled not to exceed 20 °C. After the dripping process, stirred to continue the reaction at room temperature, monitored by TLC. After the end of the reaction, ethyl acetate (20L) and water (20L) were added and stirred, then the solution was separated. The organic layer was washed with saturated sodium chloride (5L × 2) and separated. The aqueous layer was extracted once with ethyl acetate (10L). The organic layers were combined, added with activated carbon (500g) at room temperature and stirred for 1 hour. After filtration, the filtrate was concentrated to produce compound of Formula 10, which was a yellowish thick substance (2.81kg). The product did not need for further purification and was directly put into the next reaction.

¹H NMR (500 MHz, Chloroform-*d*) δ 7.81 - 7.71 (m, 2H), 7.65 - 7.53 (m, 2H), 7.40 (td, *J* = 7.5, 2.4 Hz, 2H), 7.31 (t, *J* = 7.4 Hz, 2H), 5.17 (s, 1H), 5.14 (s, 1H), 4.93 - 4.51 (m, 1H), 4.49 (dd, *J =* 10.7, 6.7 Hz, 1H), 4.41 (dd, *J* = 10.7, 6.5 Hz, 1H), 4.25 (t, *J =* 6.5 Hz, 1H), 3.92 (s, 1H), 3.76 (s, 1H), 2.85 - 2.54 (m, 3H), 1.87 - 1.74 (m, 1H), 1.73 - 1.58 (m, 1H), 1.57 - 1.33 (m, 2H).

¹³C NMR (126 MHz, Chloroform-*d*) δ 155.8, 144.1, 141.5, 141.4, 139.8, 127.73, 127.70, 127.15, 127.10, 125.09, 125.06, 120.02, 119.99, 114.4, 68.4, 67.5, 54.3, 47.5, 37.9, 33.3, 27.7, 24.2.

HRMS (m/z): calc. for C₂₃H₂₅ClNO₃ [M+H]⁺= 398.1523 ; found, 398.1530

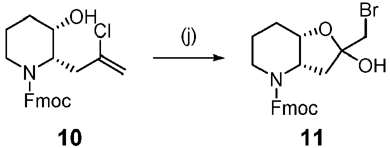

Step (j): Compound of Formula 10 (2.81kg, 5.69mol), acetonitrile (10L) and water (5L) were added into a 50L reaction kettle at room temperature, stirred and cooled to 0-5 °C. N-bromosuccinimide (1.01kg, 5.69mol) was added into the kettle in batches, and the temperature was controlled not to exceed 5 °C. After it was completed, stirred to continue the reaction at 0-5 °C and the reaction was monitored by HPLC. After the end of the reaction, 10% sodium sulfite solution (10L) was added and stirred for 0.5h. Ethyl acetate (10L × 2) was added for extraction and separation. The organic layer is washed with saturated sodium bicarbonate (5L) and saturated sodium chloride (5L× 2) and separated. The organic layer was added with activated carbon (320g) and stirred at room temperature for 1 hour, then anhydrous magnesium sulfate was added and stirred for 0.5 hours. After filtration, the filtrate was concentrated under reduced pressure to obtain compound of Formula 11, which was a dense substance (2.85 kg). The product was directly put into the next reaction without further purification.

Note: in step (i), the yield exceeded the theoretical value. Therefore, according to the 100% yield, the mole number of the compound of Formula 10 was 5.69 mol.

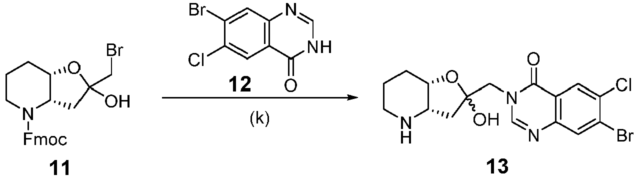

Step (k): Compound of Formula 12 (1.40 kg, 5.41 mol), lithium hydroxide (0.15 kg, 6.26 mol) and N, N-dimethylformamide (28 L) were added into a 100 L reactor at room temperature, and then the mixture was stirred at room temperature for 1 hour, then cooled to 0-5 °C. Compound of Formula 11 (2.85 kg, 5.69 mol) was dissolved in N, N-dimethylformamide (2.8 L) and then added dropwise into the reactor with temperature controlled not to exceed 5 °C. the dripping process completed within 4-5 hours. The reaction was monitored by HPLC. After the reaction was completed, diethylamine (1 L) was added into the reactor and stirred at 0°C-5 °C. The reaction was monitored by HPLC. After the reaction was completed, water (25L) and ethyl acetate (30L) were added into the reactor, then the liquid was separated by stirring. The aqueous layer was extracted by ethyl acetate (20L× 3). The organic layers were combined and concentrated at 50 °C under reduced pressure. The concentrated residue was added with 85% lactic acid solution (1.5kg), stirred at room temperature for 1 hour, and then added with methyl tert- butyl ether (10L × 2) for extraction. The aqueous layer was added with potassium carbonate to adjust to pH 8-9. Then ethyl acetate (15L × 3) was added and the organic layer was combined and concentrated at 45-50 °C under reduced pressure to produce the crude product, which was added with ethyl acetate (8L) and stirred at room temperature for 0.5h. After filtration, the filter cake was vacuum dried to obtain Compound of Formula 13 (1.84kg, 3-step yield 78%), which was a white solid.

¹H NMR (400 MHz, Chloroform-*d*) δ 8.32 (s, 1H), 8.26 (s, 1H), 7.98 (s, 1H), 4.34 (d, *J=* 13.9 Hz, 1H), 4.16 (d, *J =* 13.9 Hz, 1H), 3.88 (t, *J =* 3.1 Hz, 1H), 3.29 (t, *J =* 3.4 Hz, 1H), 2.97 (d, *J =* 10.9 Hz, 1H), 2.52 (t, *J=* 11.8 Hz, 1H), 2.10 (d, *J=* 15.1 Hz, 1H), 2.03 (dd, *J* = 13.1, 3.7 Hz, 2H), 1.83 (d, *J=* 13.2 Hz, 1H), 1.81 - 1.72 (m, 1H), 1.54 (ddt, *J* = 15.0, 12.0, 3.4 Hz, 2H).

¹³C NMR (126 MHz, Chloroform-*d*) δ 160.1, 149.5, 147.2, 133.4, 132.7, 129.4, 127.9, 122.1, 105.3, 78.0, 55.8, 50.3, 44.67, 43.7, 26.9, 20.2.

HRMS (m/z): calc. for C₁₆H₁₈BrClN₃O₃ [M+H]⁺ = 414.0220/416.0200 ; found, 414.0216/416.0197

Note: in step (i) and (j), the calculated yield exceeded the theoretical value. Therefore, according to the 100% yield, the mole number of compound of Formula 11 was 5.69 mol.

### Synthesis of halofuginone (compound of Formula 1)

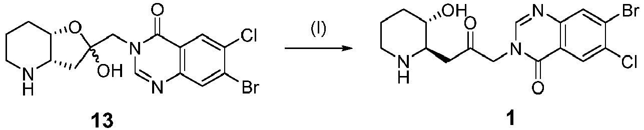

Step (l): Compound of Formula 13 (1.84kg, 4.43mol) and anhydrous ethanol (20L) were added into a 50L reaction kettle at room temperature, stirred and heated to reflux reaction, and the reaction was monitored by HPLC. After the reaction, the reaction liquid was cooled to 55-60 °C. After vacuum filtration, the filter cake was washed with ethanol and vacuum dried to obtain Compound of Formula 1, which was a white solid (1.31 kg, yield 71.2%, HPLC purity 98.6%).

¹H NMR (400 MHz, DMSO-*d*₆) δ 8.23 (s, 1H), 8.22 (s, 1H), 8.15 (s, 1H), 4.99 (d, *J =* 2.8 Hz, 2H), 4.79 (d, *J =* 5.8 Hz, 1H), 2.98 (dt, *J* = 15.3, 4.7 Hz, 2H), 2.78 (d, *J =* 12.3 Hz, 1H), 2.64 (td, *J =* 8.9, 3.8 Hz, 1H), 2.44 (dd, *J* = 15.5, 8.7 Hz, 1H), 2.36 (td, *J =* 12.1, 2.7 Hz, 1H), 1.95 - 1.83 (m, 1H), 1.56 (dt, *J* = 13.3, 3.2 Hz, 1H), 1.34 (qt, *J =* 12.4, 3.7 Hz, 1H), 1.28 - 1.13 (m, 1H).

¹³C NMR (126 MHz, DMSO-*d*₆) δ 200.7, 158.6, 149.5, 147.2, 132.4, 131.8, 128.4, 126.8, 121.7, 66.7, 56.2, 54.4, 43.0, 30.5, 20.1.

HRMS (m/z): calc. for C₁₆H₁₈BrClN₃O₃ [M+H]⁺ = 414.0220/416.0200 ; found, 414.0214/416.0195

### Embodiment 2 Synthesis of racemic halofuginone

### Preparation of intermediate compound of Formula 13

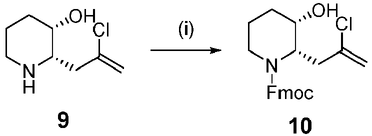

Step (i): Compound of Formula 9 (0.7kg, 3.98mol), 1,4-dioxane (3.5L), water (3.5L), sodium carbonate (0.63kg, 5.98mol) were added into a 50L reaction kettle at room temperature, stirred and cooled to 5-10 °C. Chloroformate-9-fluorene methyl ester (1.03kg, 3.98mol) was dissolved in 1,4-dioxane (1.4L) and then added dropwise into the reaction kettle, with the temperature controlled not to exceed 20 °C. After the dripping process, stirred to continue the reaction at room temperature, monitored by TLC. After the end of the reaction, ethyl acetate (15L) and water (15L) were added and stirred, then the solution was separated. The organic layer was washed with saturated sodium chloride (3L × 2) and separated. The aqueous layer was extracted once with ethyl acetate (8L). The organic layers were combined, added with activated carbon (300g) at room temperature and stirred for 1 hour. After filtration, the filtrate was concentrated to produce compound of Formula 10, which was a yellowish thick substance(1.81kg). The product did not need further purification and was directly put into the next reaction.

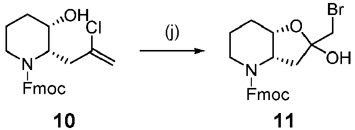

Step (j): Compound of Formula 10 (1.81kg, 3.98mol), acetonitrile (7L) and water (3.5L) were added into a 50L reaction kettle at room temperature, stirred and cooled to 0-5 °C. N-bromosuccinimide (0.71kg, 3.98mol) was added into the kettle in batches, and the temperature was controlled not to exceed 5 °C. After it was completed, stirred to continue the reaction at 0-5 °C and monitored by HPLC. After the reaction, 10% sodium sulfite solution (7L) was added and stirred for 0.5h. Ethyl acetate (7L × 2) was added to extraction and separation. The organic layer is washed with saturated sodium bicarbonate (3L) and saturated sodium chloride (3L× 2) and separated. The organic layer was added with activated carbon (200g) and stirred at room temperature for 1 hour, then anhydrous magnesium sulfate was added and stirred for 0.5 hours. After filtration, the filtrate was concentrated under reduced pressure to obtain compound of Formula 11, which was a dense substance (1.85 kg). The product was directly put into the next reaction without further purification.

Note: in step (i), the yield exceeded the theoretical value. Therefore, according to the 100% yield, the mole number of the compound of Formula 10 was 3.98 mol.

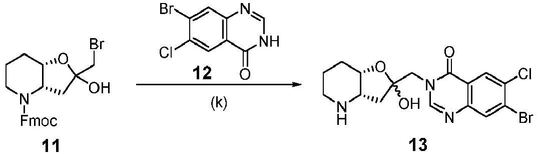

Step (k): Compound of Formula 12 (0.98kg, 3.79mol), lithium hydroxide (100g, 4.38mol) and N, N-dimethylformamide (20 L) were added into a 100 L reactor at room temperature, and then the mixture was stirred at room temperature for 1 hour, then cooled to 0-5 °C. Compound of Formula 11 (1.85kg, 3.98mol) was dissolved in N, N-dimethylformamide (2 L) and then added dropwise into the reactor with temperature controlled not to exceed 5 °C, the dripping process completed within 4-5 hours. The reaction was monitored by HPLC. After the reaction was completed, diethylamine (0.7 L) was added into the reactor and stirred at 0-5 °C. The reaction was monitored by HPLC. After the reaction was completed, water (16L) and ethyl acetate (20L) were added into the reactor, then the liquid was separated by stirring. The aqueous layer was extracted by ethyl acetate (15L× 3). The organic layers were combined and concentrated at 50 °C. The concentrated residue was added with 85% lactic acid solution (1.05kg), stirred at room temperature for 1 hour, and then added with methyl tert- butyl ether (8L × 2) for extraction. The aqueous layer was added with potassium carbonate to adjust to pH 8-9. Then ethyl acetate (10L × 3) was added and the organic layer was combined and concentrated at 45-50 °C under reduced pressure to produce the crude product, which was added with ethyl acetate (5L) and stirred at room temperature for 0.5h. After filtration, the filter cake was vacuum dried to obtain Compound of Formula 13 (1.31kg, 3-step yield 79%), which was a white solid.

Note: in step (i) and (j), the yield exceeded the theoretical value. Therefore, according to the 100% yield, the mole number of the compound of Formula 11 was 3.98 mol.

### Synthesis of halofuginone (compound of Formula 1)

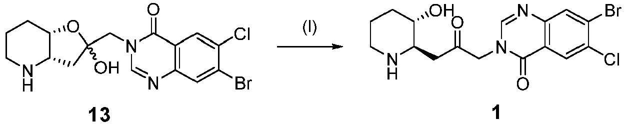

Step (l): Compound of Formula 13 (1.31kg, 3.16mol) and anhydrous ethanol (16L) were added into a 50L reaction kettle at room temperature, stirred and heated to reflux reaction, and the reaction was monitored by HPLC. After the end of the reaction, the reaction liquid was cooled to 55-60 °C. After vacuum filtration, the filter cake was washed with ethanol and vacuum dried to obrain Compound of Formula 1, which was a white solid (0.91 kg, yield 70%, HPLC purity 98.4%).

### Embodiment 3 Synthesis of racemic halofuginone

### Preparation of intermediate compound of Formula 4 (R₁=Et)

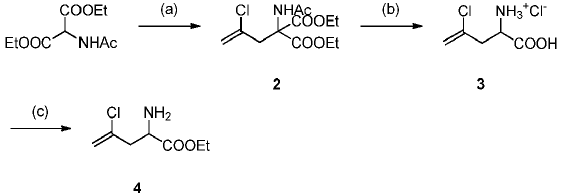

Step (a): At room temperature, diethyl acetylaminomalonic acid (2.50kg, 11.51mol), anhydrous potassium carbonate (3.18kg, 23.02mol), potassium iodide (0.38kg, 2.3mol), tetrabutylammonium bromide (0.19kg, 0.58mol) and acetonitrile (13L) were added into a 50L reaction kettle. After being stirred for 20 minutes, 2,3-dichloropropene (1.53kg,13.81mol) was added. The reaction was carried out at 85-90 °C and monitored by HPLC. After the end of the reaction, the reaction mixture was cooled to a temperature within 25 °C, and diluted hydrochloric acid was added dropwise into the reactor to neutralize the reaction mixture to pH 7-7.5. The reaction solution was left to separation, then the organic layer was concentrated under reduced pressure at 50 °C. The concentrated residue was added with ethanol water (1:10, 10L) and stirred for 1 hour for crystallization. After vacuum filtration, the filter cake was washed with water to obtain compound of Formula 2, which was a yellow solid (wet weight 5.82kg), which was not dried and directly put into the next reaction.

Step (b): At room temperature, the compound of Formula 2 (wet weight 5.82kg, 11.51mol) and hydrochloric acid solution (6mol/L, 30L, 180mol) were added into a 100L reaction kettle, stirred and heated to 100 °C for reflux reaction, and the reaction was monitored by TLC. After the end of the reaction, the reaction kettle was added with activated carbon (250g) and cooled to not to exceed 50 °C. After vacuum filtration, the filter cake was washed with water, then the filtrate was combined and concentrated under reduced pressure at 80-85 °C to remove the solvent to obtain compound of Formula 3, which was a light-yellow solid (2.05kg). The product was directly put into the next reaction without further purification.

Step (c): At room temperature, compound of Formula 3 (2.05kg, 11.02mol), diethyl carbonate (6L) and anhydrous ethanol (2L) were added into a 50L reaction kettle and stirred. Concentrated sulfuric acid (0.56kg, 5.75mol) was added dropwise and completed within 20-30 minutes. Then, heated to 85-95 °C for reflux reaction, and the reaction was monitored by HPLC. After the end of the reaction, the reaction solution was cooled to 50-60 °C and concentrated under reduced pressure, and the remaining liquid was diluted with water and cooled to not to exceed 10 °C. 30% sodium hydroxide was added dropwise to adjust the solution to pH 8-9, then moved to room temperature for extraction using dichloromethane (5L × 3). After extraction, the organic layers were combined, added with anhydrous sodium sulfate and dried. After vacuum filtration, the filtrate was concentrated at 40 °C to obtain compound of Formula 4, which was a light brown oil (1.59 kg, The total yield of 3 steps is 78%).

Note: in step (a), the product was wet without being dried, so the yield exceeded the theoretical value. Therefore, according to the 100% yield, the mole number of the compound of Formula 2 was 11.51 mol.

### Preparation of intermediate compound of Formula 6 (R₁=Et; R₂=Et; R₃=Cbz)

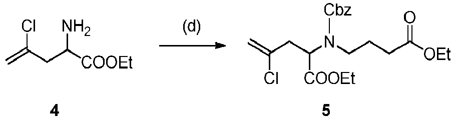

Step (d): At room temperature, compound of Formula 4 (1.50kg, 8.44mol), anhydrous sodium carbonate (2.69kg, 25.33mol), tetrabutylammonium iodide (0.31kg, 0.84mol) and toluene (4.5L) were added into a 50L reaction kettle, and toluene (3L) solution of ethyl 4-bromobutyrate (1.65kg, 8.44mol) was added after stirring for 20 minutes. The reaction was stirred at 75-80 °C and monitored by HPLC. After the end of the reaction, the temperature was cooled to 20-25 °C. Water (5L) was added and stirred for 10-15 minutes, then benzyl chloroformate (1.44kg, 8.44mol) was added dropwise and completed within about 2-3 hours. The reaction was continuously stirred at 20-25 °C and monitored by TLC. After the reaction, water (5L) and toluene (5L) were added and stirred for 0.5h to separate the solution. The organic layer was successively washed with 5% NaOH (10L), water (10L), 5% hydrochloric acid (15L) and water (10L). The organic layer was added with activated carbon (100.0g) and stirred at room temperature for 1 hour. After vacuum filtration, the filtrate was concentrated under reduced pressure at 60 °C to obtain compound of Formula 5 which was a brown oil (4.18 kg). The product was directly put into the next reaction without further purification.

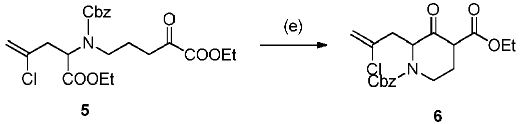

Step (e): At room temperature and under the protection of nitrogen, sodium tert-butoxide (1.62kg, 16.89 mol) and anhydrous tetrahydrofuran (19L) were added into a 100L reaction kettle and stirred, then cooled to below -5 °C. The compound of Formula 5 (4.18kg, 8.44mol) was dissolved in tetrahydrofuran (8L) and then added dropwise into the reaction kettle. The temperature during the dripping process was controlled not to exceed 0 °C and completed dripping within 4-5 hours. Continued stirring at 0-5 °C after the dripping process and the reaction was monitored by HPLC. After the end of the reaction, dilute hydrochloric acid was added to the kettle to adjust the solution to pH5-6. Ethyl acetate(5L) was added, and stirred at room temperature to divide the solution. The organic layer was washed with saturated sodium chloride (10L × 2), and the aqueous layer was extracted once with ethyl acetate (5L). The organic layer was combined and added with activated carbon (200g) and stirred for 1 hour at room temperature. After vacuum filtration, the filtrate was concentrated under reduced pressure to obtain compound of Formula 6, which was a light brown oil (2.73kg, The total yield of two steps is 85%).

Note: in step (d), the yield exceeded the theoretical value. Therefore, according to the 100% yield, the mole number of the compound of Formula 5 was 8.44 mol.

### Preparation of intermediate compound of Formula 7 (Rs=Cbz)

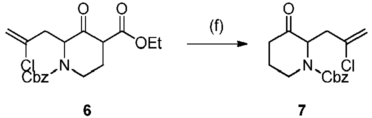

Step (f): Compound of Formula 6 (2.00kg, 5.27mol), DMF (6l), water (1L) and lithium chloride (0.22kg, 2.27mol) were added into a 50L reaction kettle at room temperature and stirred. The reaction mixture was stirred and heated to 120 °C and the reaction was monitored by HPLC. After the end of the reaction, the reaction mixture was cooled to 20-25 °C. Water (12L) and methyl tert-butyl ether (15L) were added, stirred and separated. The organic layer was washed with water (15L × 2) and separated, then the generated aqueous layer was extracted with methyl tert- butyl ether (15L). By combining the organic layers and concentrating under reduced pressure at 45 °C, compound of Formula 7 (1.52 kg, yield 94%) was obtained, which was a brown oil..

### Preparation of intermediate compound of Formula 8 (R₃=Cbz)

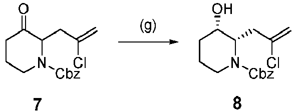

Step (g): Anhydrous ethanol (7L) and sodium borohydride (0.19kg, 4.95mol) were added into a 50L reaction kettle at room temperature and stirred, then cooled to 5-10 °C. Compound of Formula 7 (1.52kg, 4.95mol) was dissolved in anhydrous ethanol (3L) and added dropwise into the reactor under the temperature controlled not to exceed 10 °C. Keep stirring at 5- 10°C. The reaction was monitored by HPLC. After the end of the reaction, water (10L) was added dropwise into the kettle. After the dripping process, methyl tert- butyl ether (10L) was added, stirred and separated. The organic layer was washed sequentially with 10% NaOH (5L), 5% hydrochloric acid (5L×2) and Water (5L). The generated aqueous layer was extracted with methyl tert- butyl ether (15L). The organic layers were combined, and added with activated carbon (200g) and stirred at room temperature for 1 hour. After filtration, the filtrate was concentrated under reduced pressure at 45 °C to obtain the compound of Formula 8 (1.30kg, yield 85%), which was a brown oil.

### Preparation of intermediate compound of Formula 9

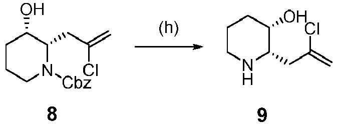

Step (h): Compound of Formula 8 (1.30kg, 4.21mol), hydrochloric acid (6mol/L, 5.61L, 33.68mol) and ethanol (6L) were added into a 50L reaction kettle at room temperature, stirred and heated for reflux reaction and the reaction was monitored by HPLC. After the reaction was completed, the reaction solution was cooled to 50-55 °C and concentrated under reduced pressure to remove ethanol. The remaining liquid was added with methyl tert- butyl ether (7L × 2), stirred and separated. The aqueous layer was adjusted to pH > 11 with 40% sodium hydroxide, and then added with ethyl acetate (10L) × 2) for extraction. The organic layers were combined, added with anhydrous magnesium sulfate and dried. After filtration, the filtrate was concentrated under reduced pressure to obtain the crude product. Acetonitrile (2L) was added to the crude product, stirred to dissolve at 70 °C, and then the product was crystallized at room temperature. After filtration and vacuum drying, compound of Formula 9 (0.35 kg, yield 48%) was obtained, which was a nearly white solid.

### Embodiment 4 Synthesis of halofuginone hydrobromate

### Synthesis of halofuginone hydrobromate (compound of Formula 1)

Hydrobromate (100g, 0.24mol) and hydrobromic acid solution (4.8%, 700ml) were added into a 1L glass reaction flask at room temperature, and stirred for 2 hours. After decompression filtration, the filter cake was washed with pure water and dried in vacuum to obtain halofuginone hydrobromate, which was a white solid (118g, yield 99%, HPLC purity 98.6%).

### Embodiment 5 Synthesis of lactate of halofuginone

### Synthesis of lactate of halofuginone (compound of Formula 1)

Halofuginone (100g, 0.24mol), pure water (400ml) and DL- lactic acid (85% aqueous solution, 25.6g, 0.24mol) were added into a 500mL glass reaction flask at room temperature. After being stirred and dissolved, the reaction solution was concentrated to remove water at 60 °C under reduced pressure. After concentration, the remaining liquid was added with ethanol (500mL) and stirred at 0 °C for crystallization. After filtration under reduced pressure, the filter cake was dried in vacuum to obtain the lactate of halofuginone, which was a white solid (84g, yield 70%, HPLC purity 99.2%).

### Embodiment 6

### Synthesis of compound of Formula (+) - 9 (dextro optically active [(+) - (2S, 3S) - 2 - (2-chloropropenyl) - 3-hydroxypiperidine])

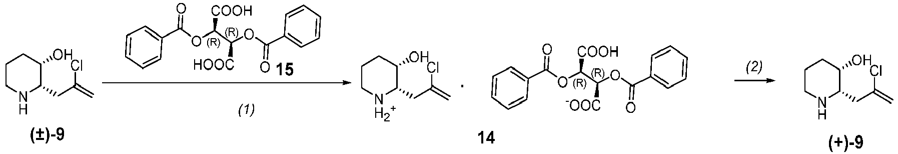

Step (1): Racemic compound of Formula 9 (140g, 0.797mol, prepared according to the method of embodiment 1) and acetonitrile (1400mL) were added into a 5L three-mouth bottle at room temperature, heated to 60 °C and stirred to dissolve. L - (-) - dibenzoyl tartaric acid (300g, 0.837mol) of Formula 15 was dissolved in acetonitrile (800mL), and then added dropwise to the reaction flask. The dripping process was completed within about 10min, continued stirring for 20-30 minutes. Then the bottle was moved to room temperature and stirred for 2 hours. After filtration, the filter was washed with acetonitrile (300mL) and drain to obtain crude compound salt, which was added with pure water and acetonitrile (1:4, 2000mL), stirred and heated to 80°C to dissolve, filtered while hot, and the filtrate was stirred at room temperature for 2 hours for crystallization. The compound of Formula 14 (136g, yield 32%) was obtained by filtration, acetonitrile leaching and drying.

Step (2): At room temperature, compound of Formula 14 (136g), pure water (700mL) and ethyl acetate (700mL) were added into a 5L three-mouth flask, stirred, and 1mol / L NaOH was added dropwise to adjust to pH 12-14. The aqueous layer was separated with ethyl acetate (700mL × 2). After extraction, organic layers were combined, added anhydrous MgSO₄ to dry. After filtration, and the filtrate was concentrated at 50 °C under reduced pressure to obtain compound (+) - 9, which wasa white solid (44g, yield 98%).

### [α]_{D}= +8.43° (c = 0.46, MeOH)

¹H NMR (500 MHz, Chloroform-*d*) δ 5.25 (d, *J =* 1.1 Hz, 1H), 5.23 (t, *J =* 1.0 Hz, 1H), 3.65 (S, 1H), 3.03 (ddt, *J* = 11.5, 4.3, 2.0 Hz, 1H), 2.88 (ddd, *J* = 7.5, 6.3, 1.4 Hz, 1H), 2.65 (td, *J* = 11.9, 2.9 Hz, 1H), 2.49 (d, *J* = 6.8 Hz, 2H)., 1.91 (dtt, *J* = 13.4, 4.0, 2.0 Hz, 1H), 1.73 (qt, *J=* 13.1, 4.3 Hz, 1H), 1.54 (tdd, *J* = 13.3, 4.7, 2.5 Hz, 1H), 1.45 (ddq, *J* = 12.9, 4.9, 2.6 Hz, 1H).

¹³C NMR (126 MHz, Chloroform-*d*) δ 139.8, 115.2, 67.1, 57.7, 47.2, 43.0, 32.2, 20.4.

HRMS (m/z): calc. for C₈H₁₅ClNO [M+H]⁺= 176.0842 ; found, 176.0837

### Embodiment 7

### Synthesis of compound of Formula (-) - 9 (I-optically active [(-) - (2R, 3R) - 2 - (2-chloropropenyl) - 3-hydroxypiperidine)]

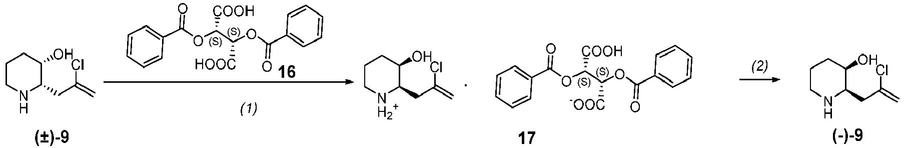

Step (1): Racemic compound of Formula 9 (14g, 79.7mmol, prepared according to the method of embodiment 1) and acetonitrile (140ml) were added into a 5L three-mouth bottle at room temperature, heated to 60 °C and stirred to dissolve. L - (-) - dibenzoyl tartaric acid (30g, 83.7mmol) of Formula 16 is dissolved in acetonitrile (80mL), and then added dropwise into the reaction flask. The dripping process was completed within about 10min, continued stirring for 20-30 minutes. Then the bottle was moved to room temperature and stirred for 2 hours. After filtration, the filter was washed with acetonitrile (30mL) and drain to obtain crude compound salt, which was added with pure water and acetonitrile (1:4, 200mL), stirred and heated to 80 °C to dissolve, filtered while hot, and the filtrate was stirred at room temperature for 2 hours for crystallization. The compound of Formula 17 (12.1g, yield 28.4%) was obtained by filtration, acetonitrile leaching and drying.

Step (2): At room temperature, compound of Formula 17(12.1g), pure water (70mL) and ethyl acetate (100mL) were added into a 5L three-mouth flask, stirred, and 1mol / L NaOH was added dropwise to adjust to pH 12-14. The aqueous layer was separated with ethyl acetate (100ml × 2). After extraction, organic layers were combined, added anhydrous MgSO4 to dry. After filtration, the filtrate was concentrated at 50 °C under reduced pressure to obtain compound (-) - 9, which was a white solid (3.7g, yield 93.1%).

### [α]_{D}= -8.43° (c = 0.46, MeOH)

¹H NMR (500 MHz, Chloroform*-d*) δ 5.25 (d, *J =* 1.1 Hz, 1H), 5.23 (t, *J =* 1.0 Hz, 1H), 3.65 (S, 1H), 3.03 (ddt, *J* = 11.5, 4.3, 2.0 Hz, 1H), 2.88 (ddd, *J* = 7.5, 6.3, 1.4 Hz, 1H), 2.65 (td, *J* = 11.9, 2.9 Hz, 1H), 2.49 (d, *J* = 6.8 Hz, 2H)., 1.91 (dtt, *J* = 13.4, 4.0, 2.0 Hz, 1H), 1.73 (qt, *J=* 13.1, 4.3 Hz, 1H), 1.54 (tdd, *J* = 13.3, 4.7, 2.5 Hz, 1H), 1.45 (ddq, *J* = 12.9, 4.9, 2.6 Hz, 1H).

¹³C NMR (126 MHz, Chloroform-*d*) δ 139.8, 115.2, 67.1, 57.7, 47.2, 43.0, 32.2, 20.4.

HRMS (m/z): calc. for C₈H₁₅ClNO [M+H]⁺= 176.0842 ; found, 176.0837

### Embodiment 8

### Synthesis of compound of Formula (+)-1, namely (+)-halofuginone (d- halofuginone)

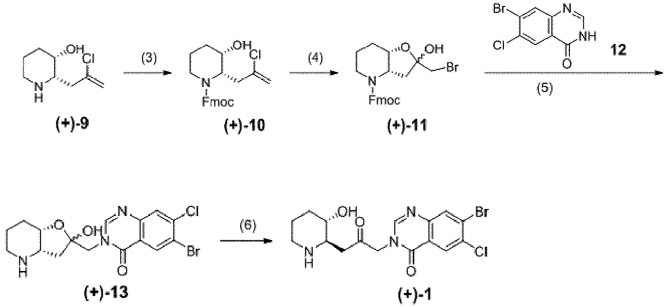

Step (3): Compound (+) - 9 (10g, 56.9mmol), 1,4-dioxane (50mL), water (50mL) and sodium carbonate (9.1g, 85.4mmol) were added into a 500mL reaction flask at room temperature, stirred and cooled to 5-10 °C. 9-fluorenylmethyl chloroformate (14.7g, 56.9mmol) was dissolved in 1,4-dioxane (20 mL) and then added dropwise to the reaction flask with the temperature controlled not to exceed 20 °C. The reaction was continued at room temperature and monitored by TLC. After the reaction, ethyl acetate (200mL) and water (200mL) were added. The reaction solution was stirred and separated. The organic layer was washed with Saturated sodium chloride (50mL × 2) and separated. The aqueous layer was extracted once with ethyl acetate (100mL). The organic layers were combined, added with activated carbon (5g) and stirred at room temperature for 1 hour. After filtration, the filtrate was concentrated under reduced pressure to obtain compound of Formula (+) - 10, which was a light yellow consistency (25.1 g, EE = 96%). The product was directly put into the next reaction without further purification.
[α]_{D}= +25.42° (c = 0.35, CHCl₃)

¹H NMR (500 MHz, Chloroform-*d*) δ 7.81 - 7.71 (m, 2H), 7.65 - 7.53 (m, 2H), 7.40 (td, *J* = 7.5, 2.4 Hz, 2H), 7.31 (t, *J* = 7.4 Hz, 2H), 5.17 (s, 1H), 5.14 (s, 1H), 4.93 - 4.51 (m, 1H), 4.49 (dd, *J =* 10.7, 6.7 Hz, 1H), 4.41 (dd, *J* = 10.7, 6.5 Hz, 1H), 4.25 (t, *J =* 6.5 Hz, 1H), 3.92 (s, 1H), 3.76 (s, 1H), 2.85 - 2.54 (m, 3H), 1.87 - 1.74 (m, 1H), 1.73 - 1.58 (m, 1H), 1.57 - 1.33 (m, 2H).

¹³C NMR (126 MHz, Chloroform-*d*) δ 155.8, 144.1, 141.5, 141.4, 139.8, 127.73, 127.70, 127.15, 127.10, 125.09, 125.06, 120.02, 119.99, 114.4, 68.4, 67.5, 54.3, 47.5, 37.9, 33.3, 27.7, 24.2.

HRMS (m/z): calc. for C₂₃H₂₅ClNO₃ [M+H]⁺= 398.1523 ; found, 398.1530

Step (4): Compound of Formula (+) - 10 (28g, 56.9mol), acetonitrile (100mL) and water (50mL) were added into a 500ml reaction flask at room temperature, stirred and cooled to 0°C-5 °C. N-bromosuccinimide (10.1g, 56.9mol) was added into the reaction flask in batches with the temperature controlled not to exceed 5 °C. After the dripping process, stirred to continue the reaction at 0-5 °C and monitored by HPLC. After the reaction, 10% sodium sulfite solution (100mL) was added and stirred for 0.5h. Then ethyl acetate (100 mL× 2) was added for extraction and separation. The organic layer was washed with saturated sodium bicarbonate (50mL) and saturated sodium chloride (50mL × 2) and separated. After the organic layer was added with activated carbon (5g) and stirred at room temperature for 1 hour, anhydrous magnesium sulfate was added and stirred for 0.5 hours. After filtration, the filtrate was concentrated under reduced pressure to obtain compound of Formula (+) - 11 [27.5g, [α]_{D}= +36.66° (c = 0.30, CHCl₃)], which was a thick substance. The product was directly put into the next step without further purification.

Step (5): Compound of Formula 12 (14 g, 54.1 mol), lithium hydroxide (1.5 g, 62.6 mol) and N, N-dimethylformamide (280 mL) were added into a 1000 mL reaction flask at room temperature, stirred at room temperature for 1 hour, and then cooled down to 0-5 °C. Compound of Formula (+) -11 (27.5g, 56.9mol) was dissolved in N, N-dimethylformamide (30mL) and then added dropwise into the reactor with the temperature controlled not to excess 5 °C, the dripping process was completed within 4-5 hours. Then stirred to continue the reaction at 0°C-5 °C and the reaction was monitored by HPLC. After the reaction was completed, diethylamine (10 mL) was added to the reactor and stirred at 0-5 °C. The reaction was monitored by HPLC. At the end of the reaction, water (250 mL) and ethyl acetate (300 mL) were added into the reactor, and the solution was separated by stirring. The aqueous layer was extracted with Ethyl acetate (200mL× 3). After extraction, the organic layers were combined and concentrated at 50 °C. The concentrated residue was added with 85% lactic acid solution (15 g), stirred at room temperature for 1 hour, and then methyl tert- butyl ether (100 mL× 2) was added for extraction. The aqueous layer was added with potassium carbonate to adjust to pH 8-9, then added with ethyl acetate (150mL × 3) for extraction. The organic layers were combined and concentrated at 45-50 °C under reduced pressure to obtain a crude product, which then was added with ethyl acetate (80mL) and stirred at room temperature for 0.5h. After filtration, the filter cake was dried in vacuum to obtain compound of Formula (+) - 13 (16.8g, 3-step yield 71%), which was a white solid.
[α]_{D}= +81.21° (c = 0.52, CHCl₃)

¹H NMR (400 MHz, Chloroform-*d*) δ 8.32 (s, 1H), 8.26 (s, 1H), 7.98 (s, 1H), 4.34 (d, *J =* 13.9 Hz, 1H), 4.16 (d, *J =* 13.9 Hz, 1H), 3.88 (t, *J =* 3.1 Hz, 1H), 3.29 (t, *J =* 3.4 Hz, 1H), 2.97 (d, *J =* 10.9 Hz, 1H), 2.52 (t, *J =* 11.8 Hz, 1H), 2.10 (d, *J=* 15.1 Hz, 1H), 2.03 (dd, *J* = 13.1, 3.7 Hz, 2H), 1.83 (d, *J=* 13.2 Hz, 1H), 1.81 - 1.72 (m, 1H), 1.54 (ddt, *J* = 15.0, 12.0, 3.4 Hz, 2H).

¹³C NMR (126 MHz, Chloroform-*d*) δ 160.1, 149.5, 147.2, 133.4, 132.7, 129.4, 127.9, 122.1, 105.3, 78.0, 55.8, 50.3, 44.67, 43.7, 26.9, 20.2.

HRMS (m/z): calc. for C₁₆H₁₈BrClN₃O₃ [M+H]⁺ = 414.0220/416.0200 ; found, 414.0216/416.0197

Step (6): Compound of Formula (+) - 13 (15g, 36.17mmol) and anhydrous ethanol (150mL) were added into a 500mL reaction flask at room temperature, stirred and heated to reflux reaction which was monitored by HPLC. After the reaction, the reaction liquid was cooled to 55-60 °C. After filtration under reduced pressure, the filter cake was leached with ethanol and vacuum dried to obtain (+)- halofuginone, which was a white solid (12.1g, yield 80.6%, HPLC purity 98.5%, EE = 99.6%).
[α]_{D}= +18.52° (c = 0.53, DMSO)

¹H NMR (400 MHz, DMSO-*d*₆) δ 8.23 (s, 1H), 8.22 (s, 1H), 8.15 (s, 1H), 4.99 (d, *J* = 2.8 Hz, 2H), 4.79 (d, *J=* 5.8 Hz, 1H), 2.98 (dt, *J* = 15.3, 4.7 Hz, 2H), 2.78 (d, *J=* 12.3 Hz, 1H), 2.64 (td, *J=* 8.9, 3.8 Hz, 1H), 2.44 (dd, *J=* 15.5, 8.7 Hz, 1H), 2.36 (td, *J=* 12.1, 2.7 Hz, 1H), 1.95 - 1.83 (m, 1H), 1.56 (dt, *J* = 13.3, 3.2 Hz, 1H), 1.34 (qt, *J=* 12.4, 3.7 Hz, 1H), 1.28 - 1.13 (m, 1H).

¹³C NMR (126 MHz, DMSO-*d*₆) δ 200.7, 158.6, 149.5, 147.2, 132.4, 131.8, 128.4, 126.8, 121.7, 66.7, 56.2, 54.4, 43.0, 30.5, 20.1.

HRMS (m/z): calc. for C₁₆H₁₈BrClN₃O₃ [M+H]⁺ = 414.0220/416.0200; found, 414.0214/416.0195

## Claims

1. A synthesis method for the halofuginone intermediate with the structure shown in Formula 9, comprising the following steps: 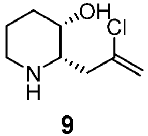
(i) alkylation reacting diethyl acetaminomalonate with 2,3-dichloropropene under the action of a base and a catalyst to form the compound of Formula 2;
(j) decarboxylation reacting the compound of Formula 2 in the presence of an acid catalyst to produce the compound of Formula 3;
(k) esterification reacting the compound of Formula 3 in the presence of an acid catalyst to produce the compound of Formula 4;
(l) nitrogen alkylation reacting the compound of Formula 4 with 4-halogenated butyrate under the action of a base and a catalyst, and then nitrogen protection reacting with amino protection reagent to produce the compound of Formula 5;
(m) Dieckmann condensation reacting the compound of Formula 5 under the action of the base to produce the compound of Formula 6;
(n) decarboxylation reacting the compound of Formula 6 in the presence of inorganic salt to produce the compound of Formula 7;
(o) reduction reacting the compound of Formula 7 under the action of a reducing agent to produce the compound of Formula 8;
(p) nitrogen deprotection reacting the compound of Formula 8 to produce the compound of Formula 9;
the reaction formulas are as follows: 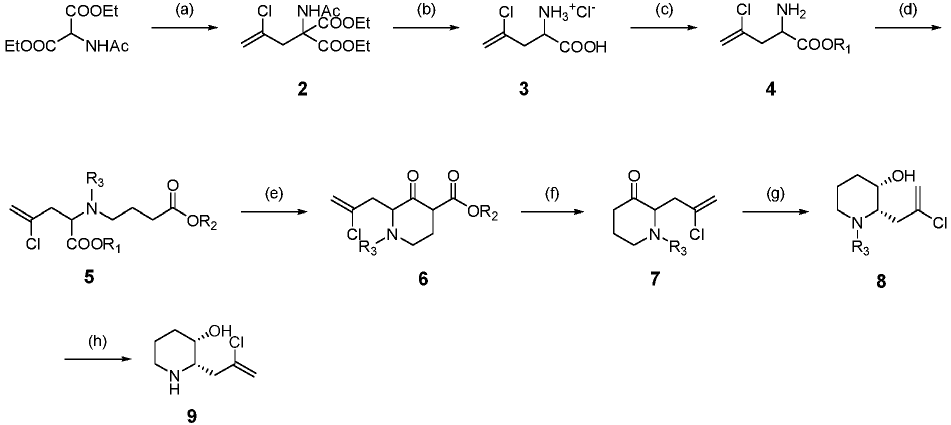
wherein,
R₁ is selected from: methyl, ethyl, propyl, isopropyl or tert -butyl;
R₂ is selected from: methyl, ethyl;
R₃ is selected from: methoxyformyl, ethoxyformyl, tert-butoxyformyl, benzyloxyformyl, trichloroethoxyformyl or benzyl.

2. The synthesis method for the halofuginone intermediate according to claim 1, wherein the base in step (a) is at least one selected from potassium carbonate, cesium carbonate, sodium carbonate, sodium hydroxide, lithium hydroxide, potassium hydroxide, sodium methoxide, sodium ethoxide, sodium tert-butoxide, potassium tert-butoxide, sodium hydride, lithium hydride and potassium hydride; and/or,
the catalyst in step (a) is the combination of quaternary ammonium salt and iodide; wherein the quaternary ammonium salt is any one selected from tetrabutylammonium bromide, tetraethylammonium bromide, tetrabutylammonium iodide and benzyl triethyl ammonium chloride; and the iodide is any one selected from sodium iodide, potassium iodide and lithium iodide; and/or,
the solvent used in the alkylation in step (a) is selected from any one of acetonitrile, methanol, ethanol, N, N-dimethylacetamide, N, N-dimethylformamide, dimethyl sulfoxide, tetrahydrofuran, 1,4-dioxane, toluene, dichloromethane and 1,2-dichloroethane; and/or,
the reaction temperature of the alkylation in step (a) is 20 °C- 120°C; and/or,
the molar ratio of diethyl acetaminomalonate, 2,3-dichloropropene, catalyst and base in step (a) is 1: (1- 2): (0.1- 0.5): (1- 3).

3. The synthesis method for the halofuginone intermediate according to claim 2, wherein the molar ratio of the quaternary ammonium salt to iodide is 1:(2-6).

4. The synthesis method for the halofuginone intermediate according to claim 1, wherein the acid in step (b) is hydrogen chloride aqueous solution, and the concentration of the hydrogen chloride aqueous solution is 5 mol / L -12mol / L; and/or,
the molar ratio of the compound of Formula 2 to hydrogen chloride is 1: (5-30) ; and/or, the acid in step (c) is selected from any one of sulfuric acid, phosphoric acid, hydrochloric acid and p-toluenesulfonic acid; and/or,
the solvent for the esterification reaction in step (c) is selected from at least one of ethanol, diethyl carbonate, dimethyl carbonate, methanol, propanol and benzyl alcohol; and/or,
the reaction temperature of the esterification reaction in step (c) is 0°C-120 °C ; and/or, the base in step (d) is selected from any one of potassium carbonate, potassium bicarbonate, cesium carbonate, sodium carbonate, sodium bicarbonate, sodium hydroxide, lithium hydroxide, potassium hydroxide, triethylamine, diisopropylethylamine, 1,8-diazabicyclo[5.4.0] undeca-7-ene; and/or,
the 4-halobutyrate in step (d) is selected from any one of 4-bromobutyrate, 4-chlorobutyrate and 4-iodobutyrate; and/or,
the catalyst in step (d) is a quaternary ammonium salt or a combination of quaternary ammonium salt and iodide, wherein the quaternary ammonium salt is selected from any one of tetrabutyl ammonium bromide, tetraethyl ammonium bromide, tetrabutyl ammonium iodide and benzyltriethylammonium chloride; and the iodide is selected from any one of sodium iodide and potassium iodide; and/or,
the solvent for the alkylation of nitrogen in step (d) is selected from any one of acetonitrile, methanol, ethanol, N, N-dimethylacetamide, N, N-dimethylformamide, dimethyl sulfoxide, tetrahydrofuran, 1,4-dioxane, toluene, dichloromethane and 1,2-dichloroethane; and/or,
the reaction temperature of the alkylation of nitrogen in step (d) is 20°C-100°C; and/or,
the molar ratio of the compound of Formula 4 in step (d), 4-halobutyrate, base and catalyst is 1:(1-1.5): (1-3): (0.01-0.2); and/or,
the amine protection reagent in step (d) is selected from any one of benzyl chloroformate, di-tert-butyl dicarbonate, methyl chloroformate, ethyl chloroformate, trichloroethyl chloroformate, benzyl bromide and benzyl chloride; and/or,
the molar ratio of the amine protection reagent in step (d) to the compound of Formula 4 is (0.8-2): 1; and/or,
the reaction temperature of the nitrogen protection reaction in step (d) is 0°C- 100°C; and/or,
the base in step (e) is selected from any one of sodium methoxide, sodium ethoxide, sodium tert-butoxide, potassium tert-butoxide, sodium hydride, lithium hydride, lithium diisopropylamide, sodium bis-(trimethylsilyl) amide, lithium bis-(trimethylsilyl) amide and potassium bis-(trimethylsilyl) amide; and/or,
the solvent for the Dieckmann condensation reaction in step (e) is selected from any one or a combination of two of tetrahydrofuran, toluene, xylene, methyl tert butyl ether, methanol and ethanol; and /or,
the molar ratio of the base in step (e) to the compound of Formula 5 is (1- 3):1; and/or,
the reaction temperature of the Dieckmann condensation reaction in step (e) is -20°C to 80°C; and/or,
the inorganic salt in step (f) is selected from any one of sodium chloride, lithium chloride, sodium bromide and lithium bromide; and /or,
the reaction solvent of the decarboxylation in step (f) is a combination of organic solvent and water, and the organic solvent in step (f) is selected from any one of dimethyl sulfoxide, sulfolane, N-methylpyrrolidone, N, N-dimethylacetamide, N, N-dimethylformamide; and/or,
the molar ratio of the inorganic salt in step (f) to the compound of Formula 6 is (1 - 3):1; and/or,
the reaction temperature of the decarboxylation reaction in step (f) is 100°C to150 °C; and/or,
the reducing agent in step (g) is selected from any one of sodium borohydride, potassium borohydride, lithium borohydride, lithium aluminum hydride, sodium bis(2-methoxyethoxy)aluminumhydride, borane, sodium amalgam and lithium tri-tert-butoxyaluminum hydride; and/or,
the solvent of the reduction reaction in step (g) is ethanol; and/or,
the molar ratio of the reducing agent in step (g) to the compound of Formula 7 is (1-2):1; and/or,
the temperature of the reduction reaction in step (g) is 0°C-10 °C.

5. The synthesis method for the halofuginone intermediate according to claim 4, wherein the volume mass ratio of the organic solvent, water and the compound of Formula 6 in step (f) is (3-5) L: (0. 1-1) L:1 kg.

6. The synthesis method for the halofuginone intermediate according to any one of claims 1-5, wherein R₃ is benzyloxyformyl, and the compound of Formula 8 undergoes nitrogen deprotection reaction under the action of acid to produce the compound of Formula 9; wherein the acid is selected from at least one of hydrochloric acid, hydrobromic acid and sulfuric acid; the solvent for the deprotection reaction in step (h) is acetic acid, water or the combination of water and alcohol, and the alcohol is any one of methanol, ethanol and isopropanol.

7. The synthesis method for the halofuginone intermediate according to any one of claims 1-5, wherein R₁ is ethyl; R₂ is ethyl; and R₃ is benzyloxyformyl.

8. A preparation method for cis-2-(2-chloropropenyl)-3-hydroxypiperidine with optical activity, comprising the following steps:
(1) in the first organic solvent, salt formation reacting the racemic cis-2-(2-chloropropenyl)-3-hydroxypiperidine with dibenzoyl tartaric acid or its derivatives to produce a precipitate, and the precipitate is recrystallized to obtain a chiral double salt;
(2) in the second organic solvent, the chiral double salt is neutralized to alkalinity with an alkaline aqueous solution to obtain a cis-2-(2-chloropropenyl)-3-hydroxypiperidine with optical activity;
the racemic cis-2-(2-chloropropenyl)-3-hydroxypiperidine has the structure as shown in Formula (±)-9; the optically active cis-2-(2-chloropropenyl)-3-hydroxypiperidine has the structure as shown in Formula (+) - 9 or Formula (-) - 9; the dibenzoyl tartaric acid or its derivative has the structure as shown in Formula 15 or Formula 16; the chiral double salt has the structure as shown in Formula 14 or Formula 17; the reaction formulas are as follows: 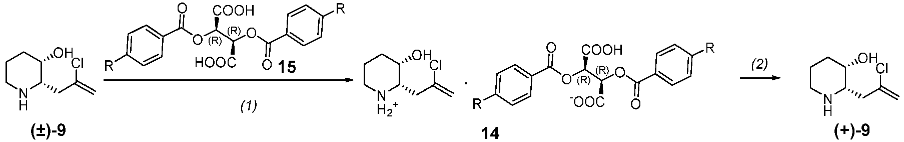 Or 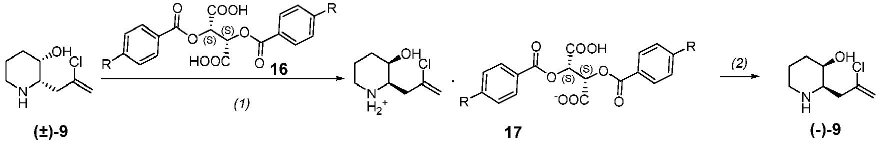
wherein each R is independently selected from: hydrogen or C₁-C₄ alkoxy.

9. The preparation method according to claim 8, wherein the dibenzoyl tartaric acid of Formula 15 or its derivative in step (1) is L- (-)- dibenzoyl tartaric acid or L- (-)-di-p-methoxybenzoyl tartaric acid; and the dibenzoyl tartaric acid of Formula 16 or its derivative is D- (+)-dibenzoyl tartaric acid or D- (+)-di-p-methoxybenzoyl tartaric acid; and / or,
the molar ratio of the racemic cis-2-(2-chloropropenyl)-3-hydroxypiperidine to dibenzoyl tartaric acid or its derivatives in step (1) is 1:(1-2); and / or,
the recrystallization is carried out in a mixed solvent of a third organic solvent to water with a volume ratio of (1-10):1; wherein the third organic solvent is selected from any one or more of ethanol, methanol, isopropanol, acetonitrile, 1,4-dioxane and acetone; and / or,
the first organic solvent in step (1) is selected from any one or more of ethanol, methanol, isopropanol, acetonitrile, dichloromethane, 1,4-dioxane, tetrahydrofuran, toluene, acetone and ethyl acetate; and / or,
the second organic solvent described in step (2) is selected from any one or more of ethyl acetate, dichloromethane and trichloromethane; and / or,
wherein the temperature of the salt formation reaction is 0°C-100 °C; and / or the temperature of recrystallization is 0°C-30 °C; and / or,
the alkaline aqueous solution in step (2) is any one of sodium hydroxide aqueous solution, potassium hydroxide aqueous solution, lithium hydroxide aqueous solution, potassium carbonate aqueous solution and sodium carbonate aqueous solution, and neutralization to pH 8-14.

10. The preparation method according to any one of claims 8 -9, wherein the synthesis method for racemic cis-2-(2-chloropropenyl)-3-hydroxypiperidine is the same as the synthesis method for the halofuginone intermediate according to any one of claims 1-7.

11. A synthesis method for racemic or optically active halofuginone, comprising the following steps:
(i) reacting the compound of Formula 9, Formula (+)-9 or Formula (-) -9 with the amino protection reagent under the action of alkali to produce a compound of Formula 10, Formula (+)-10 or Formula (-) -10;
(j) reacting the compound of Formula 10, Formula (+)-10 or Formula (-) -10 with olefin halogenation reagent and water, to produce a compound of Formula 11, Formula (+)-11 or Formula (-) -11;
(k) reacting the compound of Formula 11, Formula (+)-11 or Formula (-) -11 with the compound of Formula 12 under the action of a base; and then removing the 9-fluorenylmethoxyformyl protecting group on piperidine ring nitrogen, to produce a compound of Formula 13, Formula (+)-13 or Formula (-) -13;
(l) isomerization reacting the compound of Formula 13, Formula (+)-13 or Formula (-) -13 to produce a compound of Formula 1, Formula (+)-1 or Formula (-) -1, wherein the compound of Formula 1 is halofuginone, Formula (+)-1 or Formula (-) -1 is halofuginone with optical activity;
the reaction formulas are as follows: 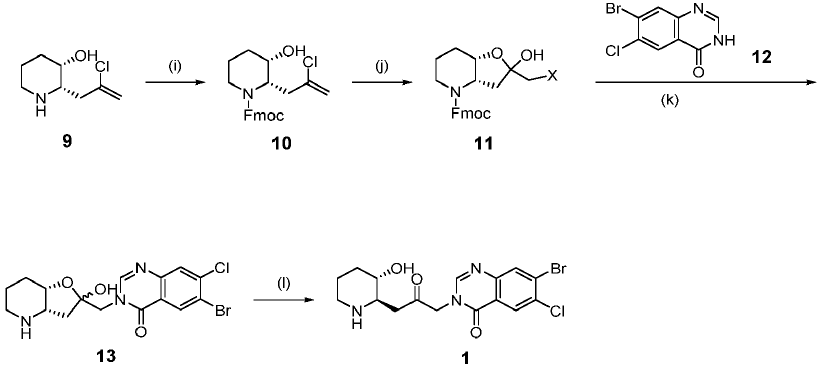 or 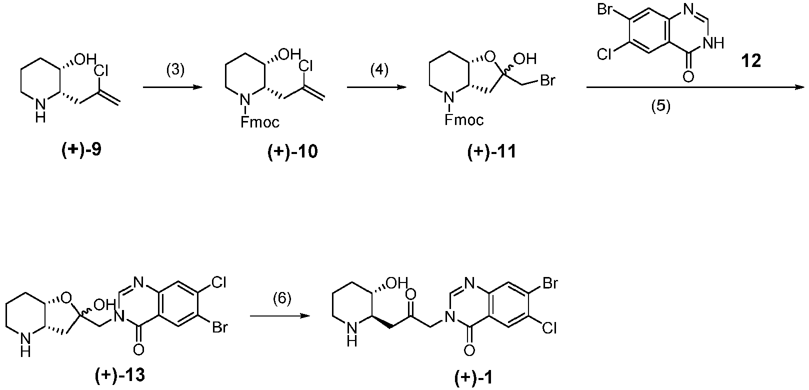 or 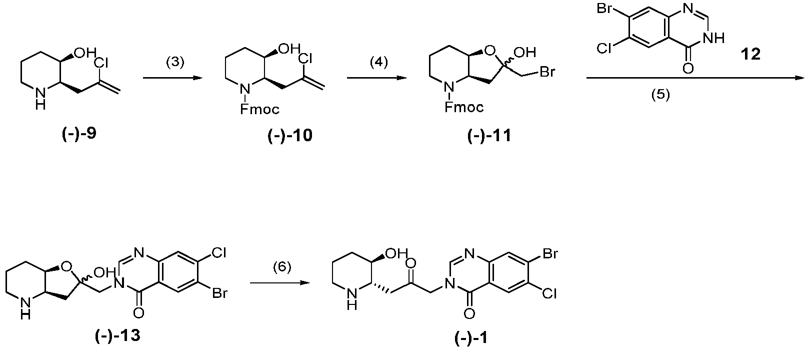
wherein, X is chlorine, bromine or iodine.

12. The synthesis method for the halofuginone according to claim 11, wherein the alkali in step (i) and step (3) is selected from any one of sodium carbonate, sodium bicarbonate, potassium carbonate and potassium bicarbonate; and/or,
the amino protection reagent in step (i) and step (3) is selected from any one of 9-fluorenylmethyl chloroformate, 9-fluorenylmethyl-1-benzotriazolyl carbonate and 9-fluorenylmethyl-n-succinimide carbonate; and/or,
the solvent in step (i) and step (3) is a combination of organic solvent and water, wherein the organic solvent is any one of 1,4-dioxane and tetrahydrofuran; and/or,
the molar ratio of the base, the amino protection reagent and the compound of Formula 9, Formula (+)-9 or Formula(-)-9 in step (i) and step (3) is (1-5):(1-2):1; and/or,
the reaction temperature in step (i) and step (3) is 0°C-20 °C;and/or,
the olefin halogenation reagent in step (j) and step (4) is selected from any one of N-bromosuccinimide, N-chlorosuccinimide, n-iodobutanimide, trichloroisocyanuric acid, 1,3-dichloro-5,5-dimethylhydantoin and 1,3-dibromo-5,5-dimethylhydantoin; and/or,
the solvent in step (j) and step (4) is selected from any one of acetonitrile, tetrahydrofuran and 1,4-dioxane; and/or,
the molar ratio of the olefin halogenation reagent to the compound of Formula 10, Formula (+)-10 or Formula (-) -10 in step (j) and step (4) is 0.9-1.2:1; and/or,
the reaction temperature in step (j) and step (4) is - 10°C to 35 °C;and/or,
the bases used in step (k) and step (5) is selected from any one of potassium carbonate, sodium carbonate, sodium hydroxide, potassium hydroxide, lithium hydroxide, cesium hydroxide, cesium carbonate, sodium methoxide, sodium ethoxide, sodium tert-butoxide, potassium tert-butoxide, sodium hydride, lithium hydride, lithium diisopropylamide, sodium bis-(trimethylsilyl) amide, lithium bis-(trimethylsilyl) amide, potassium bis-(trimethylsilyl) amide; and/or,
the solvent in step (k) and step (5) is selected from any one of acetonitrile, methanol, ethanol, N, N-dimethylacetamide, N,N-dimethylformamide, dimethyl sulfoxide, tetrahydrofuran, 1,4-dioxane, toluene, dichloromethane and 1,2-dichloroethane; and/or,
the molar ratio of the compound of Formula 12, the base and the compound of Formula 11, Formula (+) -11 or Formula (-) - 11 in step (k) and step (5) is 1:(1-2):(0.8-1.2); and/or,
the reaction temperature in step (k) and step (5) is -10°C to 25 °C; and/or,
the reaction for removing the 9-fluorenylmethoxyformyl protecting group from piperidine ring in step (k) and step (5), is carried out under the action of a secondary organic amine or a tertiary organic amine; and/or,
the reaction temperature for removing the 9-fluorenylmethoxyformyl protecting group from the piperidine ring in step (k) and step (5) is - 10°C to 25 °C;and/or,
the solvent for the isomerization reaction in steps (1) and step (6) is selected from any one or a combination of water, ethanol, methanol, n-butanol, n-propanol, tert-butanol, N,N-dimethylformamide, tetrahydrofuran and 1,4-dioxane; and/or,
the reaction temperature of the isomerization reaction in step (1) and step (6) is 50°C-80 °C.

13. The synthesis method for the halofuginone according to claim 12, wherein the secondary organic amine or the tertiary organic amine is diethylamine.

14. The synthesis method for the halofuginone according to any one of claims 11-13, wherein the preparation method for the compound of Formula (+) - 9 or Formula (-) - 9 is the same as the preparation method for cis-2-(2-chloropropenyl)-3-hydroxypiperidine with optical activity according to any one of claims 8-10.

15. The synthesis method for the halofuginone according to any one of claims 11-13, wherein the synthesis method for the compound of Formula 9 is the same as the synthesis method for the halofuginone intermediate according to any one of claims 1-7.

## Patentansprüche

1. Syntheseverfahren für das Halofuginon-Zwischenprodukt mit der in Formel 9 gezeigten Struktur, umfassend die folgenden Schritte: 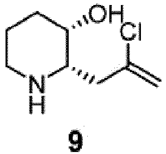
(i) Alkylierung durch Reaktion von Diethylacetaminomalonat mit 2,3-Dichlorpropen unter Einwirkung einer Base und eines Katalysators, um die Verbindung der Formel 2 zu bilden;
(j) Decarboxylierung, wobei die Verbindung der Formel 2 in Gegenwart eines sauren Katalysators umgesetzt wird, um die Verbindung der Formel 3 herzustellen;
(k) Veresterung, wobei die Verbindung der Formel 3 in Gegenwart eines sauren Katalysators umgesetzt wird, um die Verbindung der Formel 4 herzustellen;
(l) Stickstoffalkylierung, wobei die Verbindung der Formel 4 mit 4-halogeniertem Butyrat unter der Einwirkung einer Base und eines Katalysators umgesetzt wird, und anschließende Stickstoffschutzreaktion mit einem Aminoschutzreagenz, um die Verbindung der Formel 5 herzustellen;
(m) Dieckmann-Kondensation, wobei die Verbindung der Formel 5 unter Einwirkung der Base umgesetzt wird, um die Verbindung der Formel 6 herzustellen;
(n) Decarboxylierung, wobei die Verbindung der Formel 6 in Gegenwart eines anorganischen Salzes umgesetzt wird, um die Verbindung der Formel 7 herzustellen;
(o) Reduktion, wobei die Verbindung der Formel 7 unter der Wirkung eines Reduktionsmittels umgesetzt wird, um die Verbindung der Formel 8 herzustellen;
(p) Stickstoff-Entschützung, wobei die Verbindung der Formel 8 umgesetzt wird, um die Verbindung der Formel 9 herzustellen;
die Reaktionsformeln sind wie folgt: 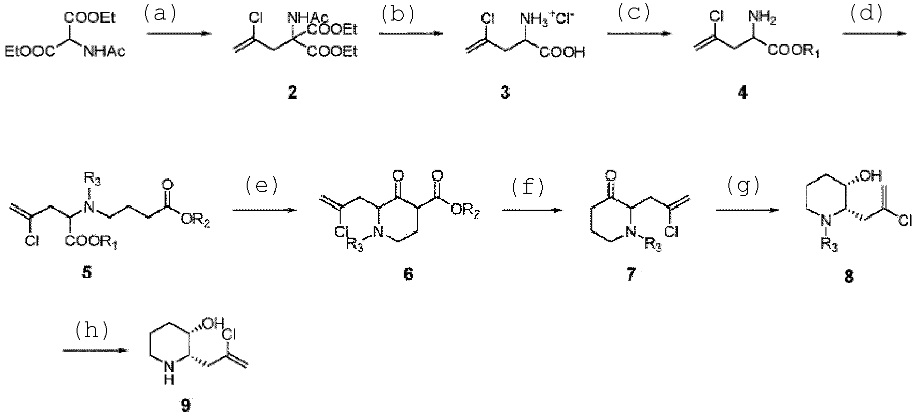
wobei,
R₁ ausgewählt ist aus: Methyl, Ethyl, Propyl, Isopropyl oder tert-Butyl;
R₂ ausgewählt ist aus: Methyl, Ethyl;
R₃ ausgewählt ist aus: Methoxyformyl, Ethoxyformyl, tert-Butoxyformyl, Benzyloxyformyl, Trichlorethoxyformyl oder Benzyl.

2. Syntheseverfahren für das Halofuginon-Zwischenprodukt nach Anspruch 1, wobei die Base in Schritt (a) mindestens eine ist, ausgewählt aus Kaliumcarbonat, Cäsiumcarbonat, Natriumcarbonat, Natriumhydroxid, Lithiumhydroxid, Kaliumhydroxid, Natriummethoxid, Natriumethoxid, Natrium-tert-Butoxid, Kaliumtert-Butoxid, Natriumhydrid, Lithiumhydrid und Kaliumhydrid; und/oder,
der Katalysator in Schritt (a) die Kombination aus quaternärem Ammoniumsalz und Iodid ist, wobei das quaternäre Ammoniumsalz ausgewählt ist aus Tetrabutylammoniumbromid, Tetraethylammoniumbromid, Tetrabutylammoniumiodid und Benzyltriethylammoniumchlorid; und das Iodid ausgewählt ist aus Natriumiodid, Kaliumiodid und Lithiumiodid; und/oder,
das Lösungsmittel, das bei der Alkylierung in Schritt (a) verwendet wird, ausgewählt ist aus Acetonitril, Methanol, Ethanol, N,N-Dimethylacetamid, N,N-Dimethylformamid, Dimethylsulfoxid, Tetrahydrofuran, 1,4-Dioxan, Toluol, Dichlormethan und 1,2-Dichlorethan; und/oder,
die Reaktionstemperatur der Alkylierung in Schritt (a) 20 °C-120 °C beträgt; und/oder,
das Molverhältnis von Diethylacetaminomalonat, 2,3-Dichlorpropen, Katalysator und Base in Schritt (a) 1: (1-2): (0,1-0,5): (1-3) beträgt.

3. Syntheseverfahren für das Halofuginon-Zwischenprodukt nach Anspruch 2, wobei das Molverhältnis des quaternären Ammoniumsalzes zu Iodid 1: (2-6) beträgt.

4. Syntheseverfahren für das Halofuginon-Zwischenprodukt nach Anspruch 1, wobei die Säure in Schritt (b) eine wässrige Chlorwasserstofflösung ist und die Konzentration der wässrigen Chlorwasserstofflösung 5 mol / 1 - 12mol / l beträgt; und/oder, das Molverhältnis der Verbindung der Formel 2 zu Chlorwasserstoff 1: (5-30) beträgt; und/oder die Säure in Schritt (c) ausgewählt ist aus Schwefelsäure, Phosphorsäure, Salzsäure und p-Toluolsulfonsäure; und/oder,
das Lösungsmittel für die Veresterungsreaktion in Schritt (c) ausgewählt ist aus mindestens einem von Ethanol, Diethylcarbonat, Dimethylcarbonat, Methanol, Propanol und Benzylalkohol; und/oder,
die Reaktionstemperatur der Veresterungsreaktion in Schritt (c) 0 °C-120 °C beträgt; und/oder die Base in Schritt (d) aus einem von Kaliumcarbonat, Kaliumbicarbonat, Cäsiumcarbonat, Natriumcarbonat, Natriumbicarbonat, Natriumhydroxid, Lithiumhydroxid, Kaliumhydroxid, Triethylamin, Diisopropylethylamin, 1,8-Diazabicyclo[5.4.0] undeca-7-en ausgewählt ist; und/oder,
das 4-Halogenbutyrat in Schritt (d) ausgewählt ist aus einem von 4-Brombutyrat, 4-Chlorbutyrat und 4-Iodbutyrat; und/oder,
der Katalysator in Schritt (d) ein quaternäres Ammoniumsalz oder eine Kombination aus quaternärem Ammoniumsalz und Iodid ist, wobei das quaternäre Ammoniumsalz ausgewählt ist aus Tetrabutylammoniumbromid, Tetraethylammoniumbromid, Tetrabutylammoniumiodid und Benzyltriethylammoniumchlorid; und das Iodid ausgewählt ist aus Natriumiodid und Kaliumiodid; und/oder,
das Lösungsmittel für die Alkylierung von Stickstoff in Schritt (d) ausgewählt ist aus Acetonitril, Methanol, Ethanol, N,N-Dimethylacetamid, N,N-Dimethylformamid, Dimethylsulfoxid, Tetrahydrofuran, 1,4-Dioxan, Toluol, Dichlormethan und 1,2-Dichlorethan; und/oder,
die Reaktionstemperatur der Alkylierung von Stickstoff in Schritt (d) 20 °C-100 °C beträgt; und/oder,
das Molverhältnis der Verbindung der Formel 4 in Schritt (d) 4-Halogenbutyrat, Base und Katalysator 1: (1-1,5): (1-3): (0,01-0,2) beträgt; und/oder,
das Aminschutzreagenz in Schritt (d) ausgewählt ist aus einem von Benzylchlorformiat, Di-tert-butyldicarbonat, Methylchlorformiat, Ethylchlorformiat, Trichlorethylchlorformiat, Benzylbromid und Benzylchlorid; und/oder,
das Molverhältnis des Aminschutzreagenzes in Schritt (d) zur Verbindung der Formel 4 (0,8-2): 1 beträgt; und/oder,
die Reaktionstemperatur der Stickstoffschutzreaktion in Schritt (d) 0 °C-100 °C beträgt; und/oder,
die Base in Schritt (e) ausgewählt ist aus Natriummethoxid, Natriumethoxid, Natrium-tert.-butoxid, Kalium-tert.-butoxid, Natriumhydrid, Lithiumhydrid, Lithiumdiisopropylamid, Natrium-bis-(trimethylsilyl)amid, Lithium-bis-(trimethylsilyl)amid und Kalium-bis-(trimethylsilyl)amid; und/oder,
das Lösungsmittel für die Dieckmann-Kondensationsreaktion in Schritt (e) ausgewählt ist aus einem oder einer Kombination aus zwei von Tetrahydrofuran, Toluol, Xylol, Methyl-tert.-butylether, Methanol und Ethanol; und /oder,
das Molverhältnis der Base in Schritt (e) zur Verbindung der Formel 5 (1-3):1 beträgt; und/oder,
die Reaktionstemperatur der Dieckmann-Kondensationsreaktion in Schritt (e) -20 °C bis 80 °C beträgt; und/oder,
das anorganische Salz in Schritt (f) ausgewählt ist aus Natriumchlorid, Lithiumchlorid, Natriumbromid und Lithiumbromid; und/oder,
das Reaktionslösungsmittel der Decarboxylierung in Schritt (f) eine Kombination aus organischem Lösungsmittel und Wasser ist und das organische Lösungsmittel in Schritt (f) ausgewählt ist aus einem von Dimethylsulfoxid, Sulfolan, N-Methylpyrrolidon, N,N-Dimethylacetamid, N,N-Dimethylformamid; und/oder,
das Molverhältnis des anorganischen Salzes in Schritt (f) zur Verbindung der Formel 6 (1 - 3):1 beträgt; und/oder,
die Reaktionstemperatur der Decarboxylierungsreaktion in Schritt (f) 100 °C bis 150 °C beträgt; und/oder,
das Reduktionsmittel in Schritt (g) ausgewählt ist aus einem von Natriumborhydrid, Kaliumborhydrid, Lithiumborhydrid, Lithiumaluminiumhydrid, Natriumbis(2-methoxyethoxy)aluminiumhydrid, Boran, Natriumamalgam und Lithiumtri-tert-butoxyaluminiumhydrid; und/oder,
das Lösungsmittel der Reduktionsreaktion in Schritt (g) Ethanol ist; und/oder,
das Molverhältnis des Reduktionsmittels in Schritt (g) zur Verbindung der Formel 7 (1-2):1 beträgt; und/oder,
die Temperatur der Reduktionsreaktion in Schritt (g) 0 °C-10 °C beträgt.

5. Syntheseverfahren für das Halofuginon-Zwischenprodukt nach Anspruch 4, wobei das Volumen-Massenverhältnis des organischen Lösungsmittels, des Wassers und der Verbindung der Formel 6 in Schritt (f) (3-5) L: (0,1-1) L:1 kg beträgt.

6. Syntheseverfahren für das Halofuginon-Zwischenprodukt nach einem der Ansprüche 1-5, wobei R₃ Benzyloxyformyl ist und die Verbindung der Formel 8 eine Stickstoffentschützungsreaktion unter Einwirkung einer Säure durchläuft, um die Verbindung der Formel 9 herzustellen; wobei die Säure ausgewählt ist aus mindestens einem von Salzsäure, Bromwasserstoffsäure und Schwefelsäure; das Lösungsmittel für die Entschützungsreaktion in Schritt (h) Essigsäure, Wasser oder die Kombination von Wasser und Alkohol ist und der Alkohol einer von Methanol, Ethanol und Isopropanol ist.

7. Syntheseverfahren für das Halofuginon-Zwischenprodukt nach einem der Ansprüche 1-5, wobei R₁ Ethyl ist; R₂ Ethyl ist; und R₃ Benzyloxyformyl ist.

8. Herstellungsverfahren für cis-2-(2-Chlorpropenyl)-3-hydroxypiperidin mit optischer Aktivität, umfassend die folgenden Schritte:
(1) in dem ersten organischen Lösungsmittel, Salzbildung durch Reaktion des racemischen cis-2-(2-Chlorpropenyl)-3-hydroxypiperidins mit Dibenzoylweinsäure oder ihren Derivaten, um einen Niederschlag zu erzeugen, und der Niederschlag wird umkristallisiert, um ein chirales Doppelsalz zu erhalten;
(2) in dem zweiten organischen Lösungsmittel wird das chirale Doppelsalz mit einer alkalischen wässrigen Lösung bis zur Alkalität neutralisiert, um ein cis-2-(2-Chlorpropenyl)-3-hydroxypiperidin mit optischer Aktivität zu erhalten;
das racemische cis-2-(2-Chlorpropenyl)-3-hydroxypiperidin die Struktur aufweist, wie in Formel (±)-9 gezeigt; das optisch aktive cis-2-(2-Chlorpropenyl)-3-hydroxypiperidin die Struktur aufweist, wie in Formel (+)-9 oder Formel (-)-9 gezeigt; die Dibenzoylweinsäure oder ihr Derivat die Struktur wie in Formel 15 oder Formel 16 gezeigt hat; das chirale Doppelsalz die Struktur wie in Formel 14 oder Formel 17 gezeigt hat; die Reaktionsformeln wie folgt sind: 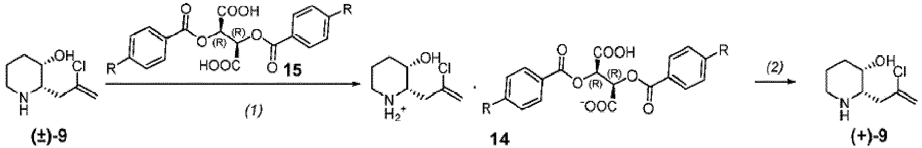 Oder 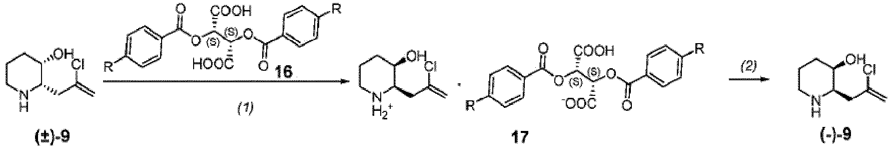 wobei jedes R unabhängig voneinander ausgewählt ist aus: Wasserstoff oder C₁-C₄ Alkoxy.

9. Herstellungsverfahren nach Anspruch 8, wobei die Dibenzoylweinsäure der Formel 15 oder ihr Derivat in Schritt (1) L- (-)-Dibenzoylweinsäure oder L- (-)-Di-p-methoxybenzoylweinsäure ist; und die Dibenzoylweinsäure der Formel 16 oder ihr Derivat D- (+)-Dibenzoylweinsäure oder D-(+)-Di-p-methoxybenzoylweinsäure ist; und/oder,
das Molverhältnis des racemischen cis-2-(2-Chlorpropenyl)-3-hydroxypiperidins zu Dibenzoylweinsäure oder ihren Derivaten in Schritt (1) 1:(1-2) beträgt; und/oder,
die Umkristallisation in einem gemischten Lösungsmittel aus einem dritten organischen Lösungsmittel und Wasser mit einem Volumenverhältnis von (1-10):1 durchgeführt wird; wobei das dritte organische Lösungsmittel aus einem oder mehreren von Ethanol, Methanol, Isopropanol, Acetonitril, 1,4-Dioxan und Aceton ausgewählt ist; und/oder,
das erste organische Lösungsmittel in Schritt (1) ausgewählt ist aus einem oder mehreren von Ethanol, Methanol, Isopropanol, Acetonitril, Dichlormethan, 1,4-Dioxan, Tetrahydrofuran, Toluol, Aceton und Ethylacetat; und/oder,
das in Schritt (2) beschriebene zweite organische Lösungsmittel ausgewählt ist aus einem oder mehreren von Ethylacetat, Dichlormethan und Trichlormethan; und/oder,
wobei die Temperatur der Salzbildungsreaktion 0 °C-100 °C beträgt; und/oder die Rekristallisationstemperatur 0 °C-30 °C beträgt; und/oder,
die alkalische wässrige Lösung in Schritt (2) eine wässrige Natriumhydroxidlösung, eine wässrige Kaliumhydroxidlösung, eine wässrige Lithiumhydroxidlösung, eine wässrige Kaliumcarbonatlösung und eine wässrige Natriumcarbonatlösung ist, und Neutralisierung auf pH 8-14.

10. Herstellungsverfahren nach einem der Ansprüche 8-9, wobei das Syntheseverfahren für racemisches cis-2-(2-Chlorpropenyl)-3-hydroxypiperidin das gleiche ist wie das Syntheseverfahren für das Halofuginon-Zwischenprodukt nach einem der Ansprüche 1-7.

11. Syntheseverfahren für racemisches oder optisch aktives Halofuginon, umfassend die folgenden Schritte:
(i) Umsetzen der Verbindung der Formel 9, Formel (+)-9 oder Formel (-)-9 mit dem Aminoschutzreagenz unter Einwirkung von Alkali, um eine Verbindung der Formel 10, Formel (+)-10 oder Formel (-) -10 herzustellen;
(j) Umsetzen der Verbindung der Formel 10, Formel (+)-10 oder Formel (-)-10 mit Olefinhalogenierungsreagenz und Wasser, um eine Verbindung der Formel 11, Formel (+)-11 oder Formel (-)-11 herzustellen;
(k) Umsetzen der Verbindung der Formel 11, der Formel (+)-11 oder der Formel (-)-11 mit der Verbindung der Formel 12 unter Einwirkung einer Base; und anschließendes Entfernen der 9-Fluorenylmethoxyformyl-Schutzgruppe am Stickstoff des Piperidinrings, um eine Verbindung der Formel 13, der Formel (+)-13 oder der Formel (-)-13 herzustellen;
(l) Isomerisierung, wobei die Verbindung der Formel 13, der Formel (+)-13 oder der Formel (-)-13 umgesetzt wird, um eine Verbindung der Formel 1, der Formel (+)-1 oder der Formel (-)-1 herzustellen, wobei die Verbindung der Formel 1 Halofuginon ist, die Formel (+)-1 oder die Formel (-)-1 Halofuginon mit optischer Aktivität ist;
die Reaktionsformeln sind wie folgt: 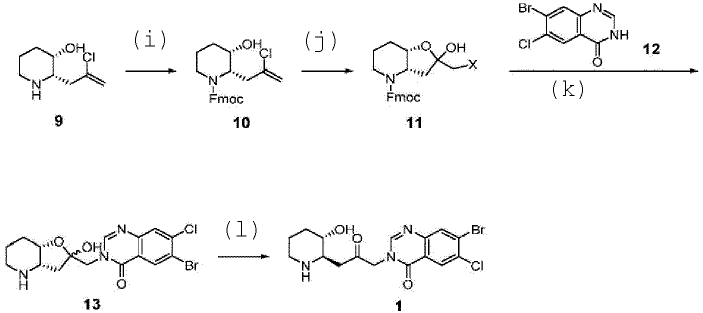 oder 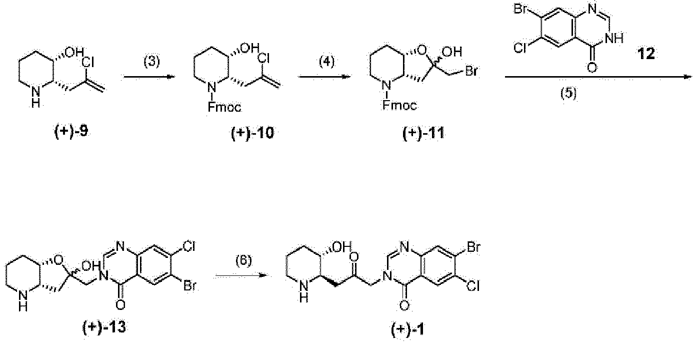 oder 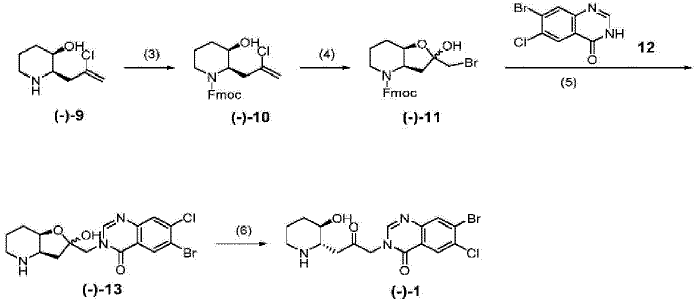 wobei X Chlor, Brom oder Jod ist.

12. Syntheseverfahren für das Halofuginon nach Anspruch 11, wobei das Alkali in Schritt (i) und Schritt (3) ausgewählt ist aus Natriumcarbonat, Natriumbicarbonat, Kaliumcarbonat und Kaliumbicarbonat; und/oder,
das Aminoschutzreagens in Schritt (i) und Schritt (3) ausgewählt ist aus 9-Fluorenylmethylchlorformiat, 9-Fluorenylmethyl-1-benzotriazolylcarbonat und 9-Fluorenylmethyl-n-succinimidcarbonat; und/oder,
das Lösungsmittel in Schritt (i) und Schritt (3) eine Kombination aus organischem Lösungsmittel und Wasser ist, wobei das organische Lösungsmittel eines von 1,4-Dioxan und Tetrahydrofuran ist; und/oder,
das Molverhältnis der Base, des Aminoschutzreagenzes und der Verbindung der Formel 9, Formel (+)-9 oder Formel(-)-9 in Schritt (i) und Schritt (3) (1-5):(1 -2):1 beträgt; und/oder, die Reaktionstemperatur in Schritt (i) und Schritt (3) 0 °C-20 °C beträgt; und/oder,
das Olefinhalogenierungsreagenz in Schritt (j) und Schritt (4) ausgewählt ist aus einem von N-Bromsuccinimid, N-Chlorsuccinimid, n-Iodbutanimid, Trichlorisocyanursäure, 1,3-Dichlor-5,5-dimethylhydantoin und 1,3 -Dibrom-5,5-dimethylhydantoin; und/oder,
das Lösungsmittel in Schritt (j) und Schritt (4) ausgewählt ist aus einem von Acetonitril, Tetrahydrofuran und 1,4-Dioxan; und/oder,
das Molverhältnis des Olefinhalogenierungsreagenzes zur Verbindung der Formel 10, Formel (+)-10 oder Formel (-)-10 in Schritt (j) und Schritt (4) 0,9-1,2:1 beträgt; und/oder,
die Reaktionstemperatur in Schritt (j) und Schritt (4) -10 °C bis 35 °C beträgt; und/oder,
die in Schritt (k) und Schritt (5) verwendeten Basen ausgewählt sind aus Kaliumcarbonat, Natriumcarbonat, Natriumhydroxid, Kaliumhydroxid, Lithiumhydroxid, Cäsiumhydroxid, Cäsiumcarbonat, Natriummethoxid, Natriumethoxid, Natrium-tert-Butoxid, Kalium-tert.-butoxid, Natriumhydrid, Lithiumhydrid, Lithiumdiisopropylamid, Natrium-bis-(trimethylsilyl)amid, Lithium-bis-(trimethylsilyl)amid, Kalium-bis-(trimethylsilyl)amid; und/oder,
das Lösungsmittel in Schritt (k) und Schritt (5) ausgewählt ist aus einem von Acetonitril, Methanol, Ethanol, N,N-Dimethylacetamid, N,N-Dimethylformamid, Dimethylsulfoxid, Tetrahydrofuran, 1,4-Dioxan, Toluol, Dichlormethan und 1,2-Dichlorethan; und/oder,
das Molverhältnis der Verbindung der Formel 12, der Base und der Verbindung der Formel 11, Formel (+)-11 oder Formel (-)-11 in Schritt (k) und Schritt (5) 1:(1-2):(0,8-1,2) beträgt; und/oder, die Reaktionstemperatur in Schritt (k) und Schritt (5) -10 °C bis 25 °C beträgt; und/oder,
die Reaktion zur Entfernung der 9-Fluorenylmethoxyformyl-Schutzgruppe vom Piperidinring in Schritt (k) und Schritt (5) unter Einwirkung eines sekundären organischen Amins oder eines tertiären organischen Amins durchgeführt wird; und/oder,
die Reaktionstemperatur zum Entfernen der 9-Fluorenylmethoxyformyl-Schutzgruppe von dem Piperidinring in Schritt (k) und Schritt (5) -10 °C bis 25 °C beträgt; und/oder, das Lösungsmittel für die Isomerisierungsreaktion in den Schritten (1) und Schritt (6) ausgewählt ist aus einem oder einer Kombination von Wasser, Ethanol, Methanol, n-Butanol, n-Propanol, tert-Butanol, N,N-Dimethylformamid, Tetrahydrofuran und 1,4-Dioxan; und/oder,
die Reaktionstemperatur der Isomerisierungsreaktion in Schritt (1) und Schritt (6) 50 °C-80 °C beträgt.

13. Syntheseverfahren für Halofuginon nach Anspruch 12, wobei das sekundäre organische Amin oder das tertiäre organische Amin Diethylamin ist.

14. Syntheseverfahren für das Halofuginon nach einem der Ansprüche 11-13, wobei das Verfahren zur Herstellung der Verbindung der Formel (+)-9 oder der Formel (-)-9 das gleiche ist wie das Verfahren zur Herstellung von cis-2-(2-Chlorpropenyl)-3-hydroxypiperidin mit optischer Aktivität nach einem der Ansprüche 8-10.

15. Syntheseverfahren für das Halofuginon nach einem der Ansprüche 11-13, wobei das Syntheseverfahren für die Verbindung der Formel 9 das gleiche ist wie das Syntheseverfahren für das Halofuginon-Zwischenprodukt nach einem der Ansprüche 1-7.

## Revendications

1. Procédé de synthèse de l'intermédiaire halofuginone avec la structure représentée dans la formule 9, comprenant les étapes suivantes : 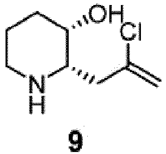
(i) alkylation par réaction d'acétaminomalonate de diéthyle avec du 2,3-dichloropropène sous l'action d'une base et d'un catalyseur pour former le composé de formule 2 ;
(j) décarboxylation par réaction du composé de formule 2 en présence d'un catalyseur acide pour produire le composé de formule 3 ;
(k) estérification par réaction du composé de formule 3 en présence d'un catalyseur acide pour produire le composé de formule 4 ;
(l) alkylation d'azote par réaction du composé de formule 4 avec du butyrate 4-halogéné sous l'action d'une base et d'un catalyseur, puis protection d'azote par réaction d'un réactif de protection aminé pour produire le composé de formule 5 ;
(m) condensation de Dieckmann par réaction du composé de formule 5 sous l'action de la base pour produire le composé de formule 6 ;
(n) décarboxylation par réaction du composé de formule 6 en présence d'un sel inorganique pour produire le composé de formule 7 ;
(o) réduction par réaction du composé de formule 7 sous l'action d'un agent réducteur pour produire le composé de formule 8 ;
(p) déprotection de l'azote par réaction du composé de formule 8 pour produire le composé de formule 9 ;
les formules de réaction sont les suivantes : 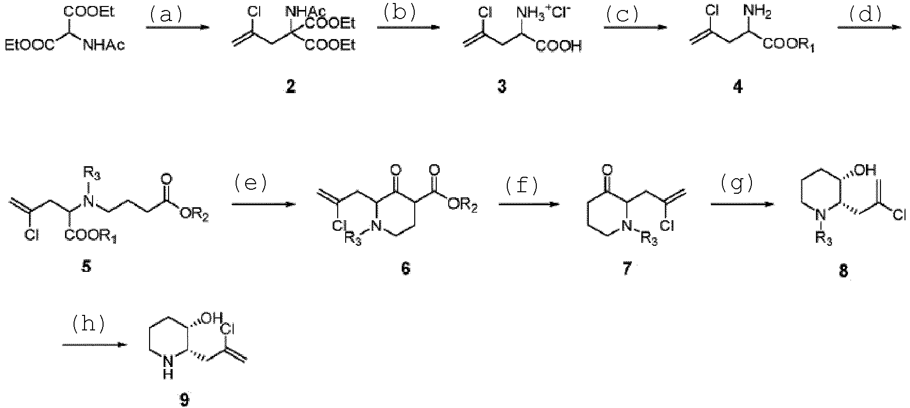 où,
R₁ est choisi parmi : méthyle, éthyle, propyle, isopropyle ou tert-butyle ;
R₂ est choisi parmi : méthyle, éthyle ;
R₃ est choisi parmi : méthoxyformyle, éthoxyformyle, tert-butoxyformyle, benzyloxyformyle, trichloroéthoxyformyle ou benzyle.

2. Procédé de synthèse de l'intermédiaire halofuginone selon la revendication 1, dans lequel la base dans l'étape (a) est au moins une base choisie parmi le carbonate de potassium, le carbonate de césium, le carbonate de sodium, l'hydroxyde de sodium, l'hydroxyde de lithium, l'hydroxyde de potassium, le méthylate de sodium, l'éthoxyde de sodium, le tert-butylate de sodium, le tert-butylate de potassium, l'hydrure de sodium, l'hydrure de lithium et l'hydrure de potassium ; et/ou,
le catalyseur dans l'étape (a) est la combinaison de sel d'ammonium quaternaire et d'iodure ; dans lequel le sel d'ammonium quaternaire est l'un quelconque choisi parmi le bromure de tétrabutylammonium, le bromure de tétraéthylammonium, l'iodure de tétrabutylammonium et le chlorure de benzyltriéthylammonium ; et l'iodure est l'un quelconque choisi parmi l'iodure de sodium, l'iodure de potassium et l'iodure de lithium ; et/ou,
le solvant utilisé dans l'alkylation dans l'étape (a) est choisi parmi l'un quelconque parmi l'acétonitrile, le méthanol, l'éthanol, le N,N-diméthylacétamide, le N,N-diméthylformamide, le diméthylsulfoxyde, le tétrahydrofurane, le 1,4-dioxane, le toluène, le dichlorométhane et 1,2-dichloroéthane ; et/ou,
la température de réaction de l'alkylation dans l'étape (a) est de 20 °C à 120 °C ; et/ou,
le rapport molaire de l'acétaminomalonate de diéthyle, du 2,3-dichloropropène, du catalyseur et de la base dans l'étape (a) est de 1: (1-2): (0,1-0,5): (1-3).

3. Procédé de synthèse de l'intermédiaire halofuginone selon la revendication 2, dans lequel le rapport molaire du sel d'ammonium quaternaire à l'iodure est de 1: (2-6).

4. Procédé de synthèse de l'intermédiaire halofuginone selon la revendication 1, dans lequel l'acide dans l'étape (b) est une solution aqueuse de chlorure d'hydrogène, et la concentration de la solution aqueuse de chlorure d'hydrogène est de 5 mol/L - 12 mol/L ; et/ou,
le rapport molaire du composé de formule 2 au chlorure d'hydrogène est de 1: (5-30) ; et/ou, l'acide dans l'étape (c) est choisi parmi l'un quelconque parmi l'acide sulfurique, l'acide phosphorique, l'acide chlorhydrique et l'acide p-toluènesulfonique ; et/ou,
le solvant pour la réaction d'estérification dans l'étape (c) est choisi parmi au moins l'un parmi l'éthanol, le carbonate de diéthyle, le carbonate de diméthyle, le méthanol, le propanol et l'alcool benzylique ; et/ou,
la température de réaction de la réaction d'estérification dans l'étape (c) est de 0 °C à 120 °C ; et/ou, la base dans l'étape (d) est choisie parmi l'un quelconque parmi le carbonate de potassium, le bicarbonate de potassium, le carbonate de césium, le carbonate de sodium, le bicarbonate de sodium, l'hydroxyde de sodium, l'hydroxyde de lithium, l'hydroxyde de potassium, la triéthylamine, la diisopropyléthylamine, le 1,8-diazabicyclo [5.4.0] undéca-7-ène ; et/ou,
le 4-halobutyrate dans l'étape (d) est choisi parmi l'un quelconque parmi le 4-bromobutyrate, le 4-chlorobutyrate et le 4-iodobutyrate ; et/ou,
le catalyseur dans l'étape (d) est un sel d'ammonium quaternaire ou une combinaison de sel d'ammonium quaternaire et d'iodure, dans lequel le sel d'ammonium quaternaire est l'un quelconque choisi parmi le bromure de tétrabutylammonium, le bromure de tétraéthylammonium, l'iodure de tétrabutylammonium et le chlorure de benzyltriéthylammonium ; et l'iodure est l'un quelconque choisi parmi l'iodure de sodium et l'iodure de potassium ; et/ou,
le solvant utilisé dans l'alkylation de l'azote dans l'étape (d) est choisi parmi l'un quelconque parmi l'acétonitrile, le méthanol, l'éthanol, le N,N-diméthylacétamide, le N,N-diméthylformamide, le diméthylsulfoxyde, le tétrahydrofurane, le 1,4-dioxane, le toluène, le dichlorométhane et 1,2-dichloroéthane ; et/ou,
la température de réaction de l'alkylation de l'azote dans l'étape (d) est de 20 °C à 100 °C ; et/ou,
le rapport molaire du composé de formule 4 dans l'étape (d), du 4-halobutyrate, de la base et du catalyseur est de 1: (1-1,5): (1-3) : (0,01-0,2) ; et/ou,
le réactif de protection d'amine dans l'étape (d) est choisi parmi l'un quelconque parmi le chloroformiate de benzyle, le dicarbonate de di-tert-butyle, le chloroformiate de méthyle, le chloroformiate d'éthyle, le chloroformiate de trichloroéthyle, le bromure de benzyle et le chlorure de benzyle ; et/ou,
le rapport molaire du réactif de protection d'amine dans l'étape (d) au composé de formule 4 est (0,8-2):1 ; et/ou,
la température de réaction de la réaction de protection de l'azote dans l'étape (d) est de 0 °C à 100 °C ; et/ou,
la base dans l'étape (e) est choisie parmi l'un quelconque parmi le méthylate de sodium, l'éthoxyde de sodium, le tert-butylate de sodium, le tert-butylate de potassium, l'hydrure de sodium, l'hydrure de lithium, le diisopropylamide de lithium, le bis-(triméthylsilyl)amide de sodium, le bis-(triméthylsilyl)amide de lithium et le bis-(triméthylsilyl)amide de potassium ; et/ou, le solvant pour la réaction de condensation de Dieckmann dans l'étape (e) est choisi parmi l'un quelconque ou une combinaison de deux parmi le tétrahydrofurane, le toluène, le xylène, le méthyl tert butyl éther, le méthanol et l'éthanol ; et/ou,
le rapport molaire de la base dans l'étape (e) au composé de formule 5 est (1-3):1 ; et/ou,
la température de réaction de la réaction de condensation de Dieckmann dans l'étape (e) est de -20 °C à 80 °C ; et/ou,
le sel inorganique dans l'étape (f) est choisi parmi l'un quelconque parmi le chlorure de sodium, le chlorure de lithium, le bromure de sodium et le bromure de lithium ; et/ou,
le solvant de réaction de la décarboxylation dans l'étape (f) est une combinaison de solvant organique et d'eau, et le solvant organique dans l'étape (f) est choisi parmi l'un quelconque parmi le diméthylsulfoxyde, le sulfolane, la N-méthylpyrrolidone, le N,N-diméthylacétamide, le N,N-diméthylformamide ; et/ou,
le rapport molaire du sel inorganique dans l'étape (f) au composé de formule 6 est (1 - 3):1 ; et/ou,
la température de réaction de la réaction de décarboxylation dans l'étape (f) est de 100 °C à 150 °C ; et/ou,
l'agent réducteur dans l'étape (g) est choisi parmi l'un quelconque parmi le borohydrure de sodium, le borohydrure de potassium, le borohydrure de lithium, l'hydrure de lithium et d'aluminium, l'hydrure de bis(2-méthoxyéthoxy)aluminium de sodium, le borane, l'amalgame de sodium et l'hydrure de tri-tert-butoxyaluminium de lithium ; et/ou,
le solvant de la réaction de réduction dans l'étape (g) est l'éthanol ; et/ou,
le rapport molaire de l'agent réducteur dans l'étape (g) au composé de formule 7 est (1-2):1 ; et/ou,
la température de la réaction de réduction dans l'étape (g) est de 0 °C à 10 °C.

5. Procédé de synthèse de l'intermédiaire halofuginone selon la revendication 4, dans lequel le rapport massique volumique du solvant organique, de l'eau et du composé de formule 6 dans l'étape (f) est (3-5) L : (0,1-1) L: 1 kg.

6. Procédé de synthèse de l'intermédiaire halofuginone selon l'une quelconque des revendications 1 à 5, où R₃ est benzyloxyformyle, et le composé de formule 8 subit une réaction de déprotection de l'azote sous l'action d'un acide pour produire le composé de formule 9 ; dans lequel l'acide est choisi parmi au moins l'un parmi l'acide chlorhydrique, l'acide bromhydrique et l'acide sulfurique ; le solvant pour la réaction de déprotection dans l'étape (h) est l'acide acétique, l'eau ou la combinaison d'eau et d'alcool, et l'alcool est l'un quelconque parmi le méthanol, l'éthanol et l'isopropanol.

7. Procédé de synthèse de l'intermédiaire halofuginone selon l'une quelconque des revendications 1 à 5, où R₁ est éthyle ; R₂ est éthyle ; et R₃ est benzyloxyformyle.

8. Procédé de préparation de la cis-2-(2-chloropropényl)-3-hydroxypipéridine à activité optique, comprenant les étapes suivantes :
(1) dans le premier solvant organique, formation de sel par réaction de la cis-2-(2-chloropropényl)-3-hydroxypipéridine racémique avec l'acide dibenzoyltartrique ou ses dérivés pour produire un précipité, et le précipité est recristallisé pour obtenir un sel double chiral ;
(2) dans le deuxième solvant organique, le sel double chiral est neutralisé jusqu'à l'alcalinité avec une solution aqueuse alcaline pour obtenir une cis-2-(2-chloropropényl)-3-hydroxypipéridine à activité optique ;
la cis-2-(2-chloropropényl)-3-hydroxypipéridine racémique a la structure représentée dans la formule (±)-9 ; la cis-2-(2-chloropropényl)-3-hydroxypipéridine optiquement active a la structure représentée dans la formule (+) - 9 ou la formule (- ) - 9 ; l'acide dibenzoyltartrique ou son dérivé a la structure représentée dans la formule 15 ou la formule 16 ; le sel double chiral a la structure représentée dans la formule 14 ou la formule 17 ; les formules de réaction sont les suivantes : 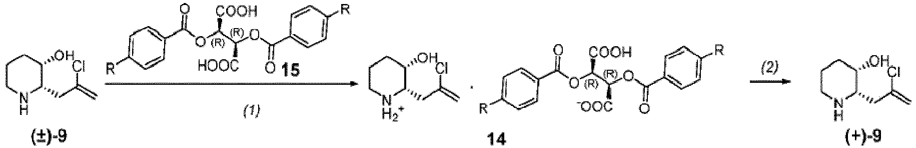 Ou 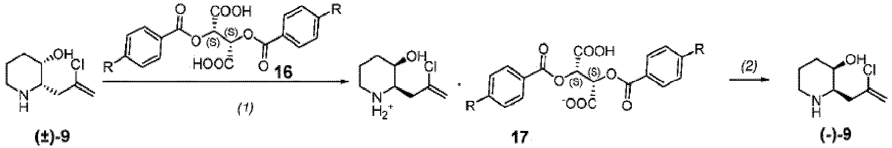
où chaque R est indépendamment choisi parmi : hydrogène ou alcoxy en C₁-C₄.

9. Procédé de préparation selon la revendication 8, dans lequel l'acide dibenzoyltartrique de formule 15 ou son dérivé dans l'étape (1) est l'acide L-(-)-dibenzoyltartrique ou l'acide L-(-)-di-p-méthoxybenzoyltartrique ; et l'acide dibenzoyltartrique de formule 16 ou son dérivé est l'acide D-(+)-dibenzoyltartrique ou l'acide D-(+)-di-p-méthoxybenzoyltartrique ; et/ou,
le rapport molaire de la cis-2-(2-chloropropényl)-3-hydroxypipéridine racémique à l'acide dibenzoyltartrique ou ses dérivés dans l'étape (1) est de 1:(1-2) ; et/ou,
la recristallisation est effectuée dans un solvant mixte d'un troisième solvant organique à l'eau avec un rapport volumique de (1-10):1 ; dans lequel le troisième solvant organique est choisi parmi un ou plusieurs parmi l'éthanol, le méthanol, l'isopropanol, l'acétonitrile, le 1,4-dioxane et l'acétone ; et/ou,
le premier solvant organique dans l'étape (1) est choisi parmi un ou plusieurs parmi l'éthanol, le méthanol, l'isopropanol, l'acétonitrile, le dichlorométhane, le 1,4-dioxane, le tétrahydrofurane, le toluène, l'acétone et l'acétate d'éthyle ; et/ou,
le deuxième solvant organique décrit dans l'étape (2) est choisi parmi un ou plusieurs parmi l'acétate d'éthyle, le dichlorométhane et le trichlorométhane ; et/ou,
dans lequel la température de la réaction de formation de sel est de 0 °C à 100 °C ; et/ou la température de recristallisation est de 0 °C à 30 °C ; et/ou,
la solution aqueuse alcaline dans l'étape (2) est l'une quelconque parmi une solution aqueuse d'hydroxyde de sodium, une solution aqueuse d'hydroxyde de potassium, une solution aqueuse d'hydroxyde de lithium, une solution aqueuse de carbonate de potassium et une solution aqueuse de carbonate de sodium, et une neutralisation à pH 8-14.

10. Procédé de préparation selon l'une quelconque des revendications 8 à 9, dans lequel le procédé de synthèse de la cis-2-(2-chloropropényl)-3-hydroxypipéridine racémique est le même que le procédé de synthèse de l'intermédiaire halofuginone selon l'une quelconque des revendications 1 à 7.

11. Procédé de synthèse d'halofuginone racémique ou optiquement active, comprenant les étapes suivantes :
(i) faire réagir le composé de formule 9, de formule (+)-9 ou de formule (-)-9 avec le réactif de protection amino sous l'action d'un alcali pour produire un composé de formule 10, de formule (+)-10 ou de formule (-)-10 ;
(j) faire réagir le composé de formule 10, de formule (+)-10 ou de formule (-)-10 avec un réactif d'halogénation d'oléfine et de l'eau, pour produire un composé de formule 11, de formule (+)-11 ou de formule (-)- 11 ;
(k) faire réagir le composé de formule 11, de formule (+)-11 ou de formule (-)-11 avec le composé de formule 12 sous l'action d'une base ; puis élimination du groupe protecteur 9-fluorénylméthoxyformyle sur l'azote du cycle pipéridine, pour produire un composé de formule 13, de formule (+)-13 ou de formule (-)-13 ;
(l) isomérisation par réaction du composé de formule 13, de formule (+)-13 ou de formule (-)-13 pour produire un composé de formule 1, de formule (+)-1 ou de formule (-)-1, dans lequel le composé de formule 1 est l'halofuginone, de formule (+)-1 ou de formule (-)-1 est l'halofuginone avec une activité optique ; les formules de réaction sont les suivantes : 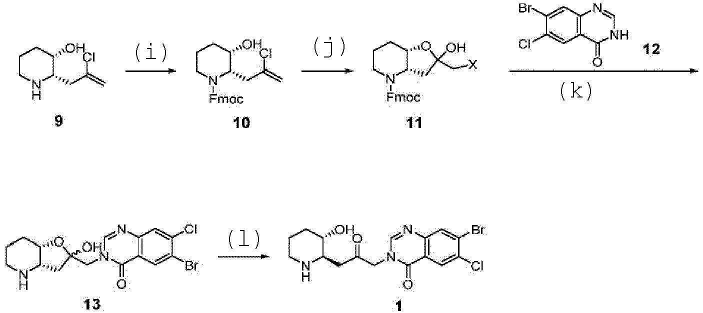 ou 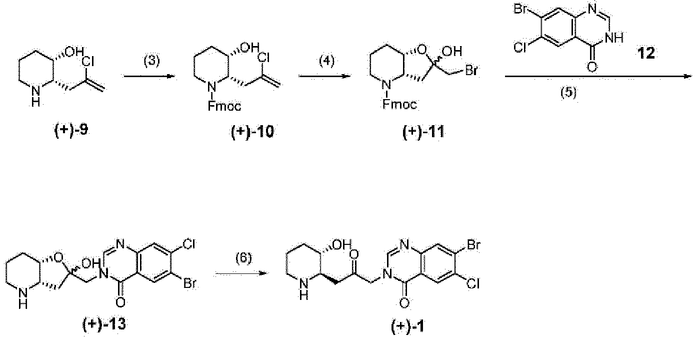 ou 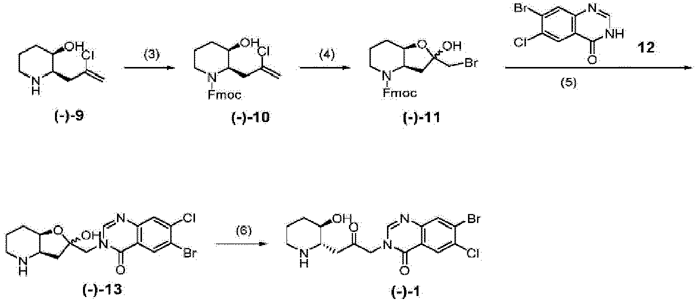 où X est le chlore, le brome ou l'iode.

12. Procédé de synthèse de l'halofuginone selon la revendication 11, dans lequel l'alcali dans l'étape (i) et l'étape (3) est choisi parmi l'un quelconque parmi le carbonate de sodium, le bicarbonate de sodium, le carbonate de potassium et le bicarbonate de potassium ; et/ou,
le réactif de protection amino dans l'étape (i) et l'étape (3) est choisi parmi l'un quelconque parmi le chloroformiate de 9-fluorénylméthyle, le carbonate de 9-fluorénylméthyl-1-benzotriazolyle et le carbonate de 9-fluorénylméthyl-n-succinimide ; et/ou,
le solvant dans l'étape (i) et l'étape (3) est une combinaison de solvant organique et d'eau, dans lequel le solvant organique est l'un quelconque parmi le 1,4-dioxane et le tétrahydrofurane ; et/ou,
le rapport molaire de la base, du réactif de protection aminé et du composé de formule 9, de formule (+)-9 ou de formule(-)-9 dans l'étape (i) et l'étape (3) est (1-5):(1-2):1 ; et/ou,
la température de réaction dans l'étape (i) et l'étape (3) est de 0 °C à 20 °C ; et/ou,
le réactif d'halogénation d'oléfine dans l'étape (j) et l'étape (4) est choisi parmi l'un quelconque parmi le N-bromosuccinimide, le N-chlorosuccinimide, le n-iodobutanimide, l'acide trichloroisocyanurique, la 1,3-dichloro-5,5-diméthylhydantoïne et la 1,3 -dibromo-5,5-diméthylhydantoïne ; et/ou,
le solvant dans l'étape (j) et l'étape (4) est choisi parmi l'un quelconque parmi l'acétonitrile, le tétrahydrofurane et le 1,4-dioxane ; et/ou,
le rapport molaire du réactif d'halogénation d'oléfine au composé de formule 10, de formule (+)-10 ou de formule (-)-10 dans l'étape (j) et l'étape (4) est de 0,9-1,2:1 ; et/ou,
la température de réaction dans l'étape (j) et l'étape (4) est de -10 °C à 35 °C ; et/ou,
les bases utilisées à l'étape (k) et à l'étape (5) sont choisies parmi l'un quelconque parmi le carbonate de potassium, le carbonate de sodium, l'hydroxyde de sodium, l'hydroxyde de potassium, l'hydroxyde de lithium, l'hydroxyde de césium, le carbonate de césium, le méthylate de sodium, l'éthoxyde de sodium, le tert-butylate de sodium, le tert-butylate de potassium, l'hydrure de sodium, l'hydrure de lithium, le diisopropylamide de lithium, le bis-(triméthylsilyl)amide de sodium, le bis-(triméthylsilyl)amide de lithium, le bis-(triméthylsilyl)amide de potassium ; et/ou,
le solvant dans l'étape (k) et l'étape (5) est choisi parmi l'un quelconque parmi l'acétonitrile, le méthanol, l'éthanol, le N,N-diméthylacétamide, le N,N-diméthylformamide, le diméthylsulfoxyde, le tétrahydrofurane, le 1,4-dioxane, le toluène, le dichlorométhane et le 1,2-dichloroéthane ; et/ou, le rapport molaire du composé de formule 12, de la base et du composé de formule 11, de formule (+)-11 ou de formule (-)-11 dans l'étape (k) et l'étape (5) est de 1: (1-2):(0,8-1,2) ; et/ou,
la température de réaction dans l'étape (k) et l'étape (5) est de -10 °C à 25 °C ; et/ou,
la réaction d'élimination du groupe protecteur 9-fluorénylméthoxyformyle du cycle pipéridine dans l'étape (k) et l'étape (5), est réalisée sous l'action d'une amine organique secondaire ou d'une amine organique tertiaire ; et/ou,
la température de réaction pour éliminer le groupe protecteur 9-fluorénylméthoxyformyle du cycle pipéridine dans l'étape (k) et l'étape (5) est de -10 °C à 25 °C ; et/ou,
le solvant pour la réaction d'isomérisation dans les étapes (1) et l'étape (6) est choisi parmi l'un quelconque ou une combinaison d'eau, d'éthanol, de méthanol, de n-butanol, de n-propanol, de tert-butanol, de N,N-diméthylformamide, de tétrahydrofurane et de 1,4-dioxane ; et/ou,
la température de réaction de la réaction d'isomérisation dans l'étape (1) et l'étape (6) est de 50 °C à 80 °C.

13. Procédé de synthèse de l'halofuginone selon la revendication 12, dans lequel l'amine organique secondaire ou l'amine organique tertiaire est la diéthylamine.

14. Procédé de synthèse de l'halofuginone selon l'une quelconque des revendications 11 à 13, dans lequel le procédé de préparation du composé de formule (+)-9 ou de formule (-)-9 est le même que le procédé de préparation de cis-2-(2-chloropropényl)-3-hydroxypipéridine à activité optique selon l'une quelconque des revendications 8 à 10.

15. Procédé de synthèse pour l'halofuginone selon l'une quelconque des revendications 11 à 13, dans lequel le procédé de synthèse pour le composé de formule 9 est le même que le procédé de synthèse pour l'intermédiaire halofuginone selon l'une quelconque des revendications 1 à 7.
